# EUROPEAN PATENT APPLICATION

(11) **EP 1 844 790 A2**
(43) Date of publication of application: **17.10.2007**
(21) Application number: 07251592.7
(22) Date of filing: 13.04.2007
(51) Int. Cl.: A61K 39/145, A61K 39/104, C12N 15/44, C07K 14/11, C07K 14/21

(54) **Using a reverse genetic engineering platform to produce protein vaccinces and protein vaccine of avian influenza virus**

(30) Priority: 14.04.2006 US 791880 P; 09.04.2007 US 783300
(71) Applicant: Healthbanks Biotech Co. Ltd., Da-an Taipei City (TW)
(72) Inventor: Liao, Chao-Wei, Shin-Chu City 300 (TW); Chang, Hsiu-Kang, Da-an District Taipei City (TW); Hwang, KinKai, Belle Mead New Jersey 08502 (US)
(74) Representative: Tranter, Andrew David

(57) **Abstract**

The present invention relates to a preparation method of protein vaccines, and comprises the steps of: (a) providing at least one amino acid sequence of an epitope of a target antigen protein; (b) converting the amino acid sequence into a nucleic acid sequence and modifying the codons; (c) synthesising a plurality of primers of the modified nucleic acid sequence; (d) synthesising the modified nucleic acid sequence *in vitro;* (e) inserting the synthesized fragment of the modified nucleic acid sequence into a plasmid; (f) transforming the plasmid into a host cell to produce the modified nucleic acid encoded epitope peptide; and (g) collecting and purifying the produced peptide.

## Description

### BACKGROUND OF THE INVENTION

### 1. Field of the Invention

The present invention is related to a method for preparing a functional protein or vaccine, particularly a method for preparing a protein subunit vaccine or a vaccinal virus strain capable of preventing or inhibiting epidemic disease.

### 2. Description of Related Art

The recent breakthroughs in the research of molecular biology, the development of various high-throughput instruments, and advanced approaches enable researchers to obtain gene sequences of various species readily. Through decoding these gene sequences which code life, scientists are able to perform research that may improve human health. Among them, one of the most important aspects of research is the R&D of functional protein and vaccines.

The conventional developing procedures of a vaccine against epidemic diseases are complicated. Moreover, these procedures are also inefficient for developing vaccines against various novel epidemic diseases. Generally speaking, the R&D work for developing a new vaccine must be conducted under a series of protection measures and precautions. The subsequent identification, classification and drug-resistance analysis must be performed after sampling and culturing of wild type virus strain from the affected subject is achieved. However, if the highly contagious strains are wrongly manipulated, they will be spread out from laboratories very easily. The possible spreading actually poses great threats not only to the researchers but also to the society as a whole.

To prevent the epidemic disease from further spreading, developing vaccines is the one of the most appropriate directions in which to proceed. However, it still requires a long period of time to tame and attenuate the virus into a vaccinal strain in time for research. For a disease that has the potential to spread so rapidly to cause a global pandemic, time is a great challenge to human beings. Likewise, due to various limitations from highly hazardous novel pathogens, the aggressive attempts to fight against these diseases are somewhat thwarted. During an outbreak, medical personnel in the affected areas are flooded by the problems of the patients, and desperately await vaccines and drugs. Hence, only limited time is left, if any, for developing vaccines and drugs. Furthermore, the people in the non-affected areas, who are afraid to contact the pathogens, can only offer and afford limited aid.

To prevent the crisis of a global outbreak of a new type Flu, developing a safe and effective vaccine is required. For safety consideration of mass-production, the newly developed reverse genetics of Flu virus strain is promising. Reverse genetics Flu virus strains are based on conventional viral strains and clone all genes into 8 vectors containing H1N1 PA, PB1, PB2, NP, M, NS2, HA, and NA, respectively. If they are co-transformed into a host cell, it is able to produce H1N1 virus bodies to enable massive production of H1N1 vaccine under appropriate culturing conditions. In 2002, Hoffmann E. used recombination approaches to recombine vectors containing genes encoding structural proteins HA and NA of H3N2, H6N1, and H9N2, and vectors containing genes encoding PA, PB 1, PB2, NP, and M to generate a novel vaccinal strain. Though the method seems to be practical, the recombination of the whole genome is potentially dangerous since it could artificially induce harmful viral strains.

According to the past studies in immunology, antigens are able to stimulate the lymphocytes carrying specific antigen receptors, induce amplification and immunity, and subsequently eliminate these antigens themselves. This is attributable to the antigen specificity of the immune system. The sites on the antigens for recognition and binding on antibodies are called "antigen determinant", or "epitope". The epitopes on an antigen usually comprise 6-8 amino acids, which can be a structure with a three-dimensional conformation.

The epitopes recognized by a T cell are epitope peptides consisting of a series of amino acids as mentioned above. They are in conjunction with MHC (major histocompatibility complex) class I/II and bind TCR (T T-cell receptors) on the cell surface of T cells when they are functioning. Each antigen typically has several epitopes, and the number of epitopes increases with the complexity of the structure, and the molecular weight of the antigens. Thus, whether the amino acid sequences of epitope peptides can be obtained from the publication, and proceeded with in vitro transcription to epitope peptides is critical to the research.

Meanwhile, to rigorously express the epitope peptides in the amplification system of the host cells, whether the host cells are able to recognize the codons encoding the recognized epitope peptides is also crucial. In addition to prior treatment, subsequent steps are necessary to proceed with mass production, purification, and isolation.

Besides epitope peptides, antigenic neutralizing zones are also the key to successful development of a vaccine. A vaccine able to induce a high neutralizing titer can effectively inhibit infection and proliferation of the virus. In addition to the above considerations, it is also important for the vaccinal compositions to be safe and innocuous, and to cause no immune disorders, immunotoxicity or allergy.

### SUMMARY OF THE INVENTION

In view of the aforementioned problems, the present invention provides a technological platform. The platform of the present invention includes steps of the most recent generation of genetic engineering technology, nucleic acid synthesis techniques, protein engineering and reverse genetics, and results in a so-called "reverse genetic engineering platform".

Using this platform, one can design and accomplish a vaccinal antigen or a modified DNA sequence of any gene encoding a target protein through the published data on the Internet, obviating the needs for contagious and hazardous biological materials. The sequence can be ligated to a DNA plasmid comprising a translocation system (for example, a pseudomonas exotoxin) or a highly antigenic sequence (e.g. the KDEL family) so as to form a fusion gene.

The fusion gene on the plasmid can produce functional proteins or a subunit vaccine in a host cell; in addition, "reverse genetics preparing novel novel vaccine RNA virus body" techniques can also be derived, leading to a safe, immuno-effective and novel Flu vaccinal virus strain. The strain is generated by inserting the determinants having antibody neutralization titration epitope in the genes of a highly contagious and hazardous virus into the corresponding loci of the original Flu H1N1 vaccine strain, which are subsequently cloned into the plasmids having similar genetic characteristics in the eight-plasmid-system used for producing vaccines, then are co-transformed with seven other plasmids containing vaccinal genes into host cells or embryos, so that these composite plasmids can synthesize novel influenza vaccine strain in host cells.

As to pathogenic and harmful diseases, a vaccine that is effective in inducing antibodies and is also safe can be rapidly developed by the above-mentioned "reverse genetic engineering platform", allowing more researchers to devote themselves to the R&D of various novel vaccines against infectious diseases so as to prevent the diseases from spreading and provide efficient and workable vaccine development techniques.

The key points of the present invention are as follow:
a. First, converting the target amino acid sequence to the corresponding nucleotide codons in order to deduce a target nucleic sequence. Because one amino acid sequence corresponds to multiple nucleotide sequences, those suitable for expression in s *E. coli* systems should be selected from literature (for example, http://www.kazusa.or.jp/codon/), and those not easily recognized and expressed by *E.coli* should be avoided. Likewise, if the sequence is to be expressed in yeasts, sequences suitable for expression in yeast systems (i.g. *Saccharomyces, or Pichia spp.)* should be selected.
b. Analyzing the restriction enzyme map of the target nucleic acid sequence by software (such as DNA strider). Then designing and linking restriction sites for restriction digestion to both termini of the target nucleic acid sequence is achieved. The sequences of the restriction sites not supposed to reside in the target sequence should be replaced by different codons encoding the same amino acids, so that those restriction sites which can be removed.
c. The regions that are possibly toxic, causing immune disorders, leading to immune toxicity or allergy in the target protein according to literatures should be modified through point mutation or deletion of its amino acid sequence if possible. For example, ultra virulence caused by basic amino acids existing in the structural proteins of the influenza virus can be attenuated by mutating them to other non-basic amino acids. By modifying the target protein in any given site, it is possible to provide design of a protein vaccine capable of inhibiting an influenza virus.
d. Of course, the modified version of the target protein should be inspected and converted to the corresponding restriction of the target gene. If any new restriction site appears in the modified target gene and causes difficulties in cloning the gene into a plasmid, it can be removed by substituting the condon with another one encoding the same amino acid.

The aforementioned points and steps are further clarified by examples given as follows. Avian flu virus belongs to a subtype of Influenza A virus, generally has a diameter about 0.08-0.12µ m, and is an RNA virus usually classified by types of HA/hemagglutinin and NA/neuraminidase on its surface. There are 15 HA subtypes and 9 NA subtypes. The human influenza viruses are usually H1N1 or H3N2, which have been pervasive for many years, so most people are immune-resistant to them. Generally, avian flu viruses are genetically distinct from human flu viruses, but cases of transmission from animal to human have been reported, such as H9, H7 and H5.

The three proteins of the capsule of A type flu virus are HA (hemmagglutinin), NA(neuraminidase) and M2, which are the major targets of recognition by antibodies of the host or anti-virus drugs. HA glycoprotein forms spikes on the surface of the virus, controls adhesion to sialoside receptors of the host cell and then enters the cell by fusion to the cell membrane. NA forms spherical spikes and catalyzes the release of viruses from infected cells, so as to spread the viruses. M2 is a membrane protein that is responsible to form an ion channel, allowing genes of the virus to be released and expressed.

A/H5N1 avian flu virus is also called "H5N1" virus, which is a novel type-A influenza virus subtype existing mainly among birds. Bird flu virus transmits continuously among birds and mutates very easily. Furthermore, the habitation areas of birds, livestock and human beings overlap considerably, so the cross-species transmission occurs easily. Pigs or humans can acquire different virus genes; for example, patients could be infected by human flu virus and bird flu virus at the same time, resulting in a "hybrid channel" of viruses and even new virus strains due to recombination. When a virus is able to infect a person and cause serious diseases, it possesses the characteristics of an outbreak-inducing flu virus strain. The looming crisis is that these kinds of viruses are prone to undergo gene flow, which could evolve to a pandemic of human-to-human transmission. Before any antibody is available to be induced in human bodies, a severe pandemic can be expected.

The present invention can derive development and application of two kinds of vaccines. The first one utilizes eight-plasmid flu system and reverse genetic engineering. First, HA structural protein is modified to a HA plasmid in which the neutralization titration regions are replaceable. Then the target sequences of the neutralization titration regions of H5N1 or other virulent novel flu virus are generated by the nucleic acid synthesis method. Finally, the synthesized fragments are inserted into replaceable HA plasmids of the H1N1 vaccine strain. The antigen composition does not include a full-length HA of the novel highly pathogenic strain H5N1, and only neutralization titration regions H5N1-HA are substituted. Thus, the properties of the virus are very similar to the H1N1 vaccine strain, so it is less probable to evolve to a highly pathogenic virus strain, endowing more safety to the manufacturing process of the vaccine. Besides, the novel flu vaccine can generate antibodies having neutralization titration after administration. Utilizing the above-mentioned method, that is, to insert the target antigen gene of the H5N1-HA neutralization titration regions into the homologous loci of the similar genes, then to employ reverse genetic engineering and eight-plasmid flu system, and to generate a vaccine strain against novel pathogens, is the best strategy for humans to fight against novel infectious diseases.

In addition to the neutralization titration regions, ELISA using non-neutralization titration regions of H5N1-HA is still able to distinguish species-specific antibodies arising from a natural infection. Thus, the ELISA system using reaction to this specific antibody after administration of the vaccine will not interfere with current detection systems in surveillance of real time situations regarding the spreading of a novel flu.

Another method of vaccine preparation of the present invention is the method to prepare a target subunit vaccine, comprising: (a) providing an amino acid sequence of at least one epitope peptide of a target antigen protein, and converting the amino acid sequence to a corresponding wild type nucleic acid sequence; (b) modifying the wild type nucleic acid sequence of the epitope to a modified nucleic acid sequence which is recognizable to a host cell and encodes the epitope peptide; (c) synthesizing primers of the modified nucleic acid sequence, wherein the primers are nucleic sequences having 5-200 nucleic acids, the primers are identical or complementary to portions of the modified nucleic acid sequence, and the 3' ends of the forward primers and the 3' ends of the reverse primers among the primers comprise sequences of 5-20 nucleic acids that are complementary to each other; (d) synthesizing the modified nucleic acid in vitro using the primers; (e) linking the synthesized nucleic acid fragments to a nucleic acid sequence having the functions of binding and translocation and a plasmid having carboxyl terminal moiety to produce a modified plasmid; (f) transforming the modified plasmid into a host cell, so as to produce the epitope peptide encoded by the modified nucleic acid; and (g) collecting and purifying the epitope peptide.

The length of the primers used in the present method are not limited. Preferably, the primers are of 5-200 nucleic acids. More preferably, the primers are of 5-80 nucleic acids.

In the method of the present invention, the target antigen genes can also be inserted into the homologous loci of the similar genes in the vaccine strain, and then reverse genetic engineering and eight-plasmid flu system are employed so as to generate a vaccine strain against novel pathogens.

In prior arts of the related field, it is known that a short peptide sequence can be used to prepare antibodies against the peptide itself, and the antibodies are able to recognize the original whole protein. Therefore, the method for preparing a subunit vaccine disclosed in the present invention is to select a segment on the epitope sequence to serve as a target synthesized peptide, without using a full-length sequence. Thus, there is no particular limitation to the sequences suitable for this method; the sequences can be mainly functional fragments, such as epitopes stimulating B-cell or T-cell immunity, or the choice of desired fragments can be based on hydrophobicity of the structure. The hydrophilic regions are more reactive to intracellular components, so it is preferable that the epitope peptides are derived from hydrophilic regions of the target antigen protein structure. The fragments used are not limited. Several fragments of choices can be joined together to form a large fragment of a peptide, a single fragment can be selected from an epitope sequence, or epitopes stimulating B-cell or T-cell immunity can be fused to form a fusion protein.

The protein structures of sequences of the present invention are not directly isolated from natural bacteria or virus, so it is necessary to synthesize and produce target proteins by using host cells. There is no particular limitation to suitable host cells, but the host cells are preferable to be microbe cells, plant cells or animal cells, and more preferable to be *E.coli* or yeasts. Besides, the target peptide (having a synthesized nucleic sequence) synthesized by host cells must encode the same target epitope peptide as in a wild type nucleic acid sequence to achieve the effects of specificity of the antigens prepared by the method disclosed in the present invention. It is advantageous for preparing an antigen vaccine having great safety and for achieving the same specificity as wild type virus antigens. Although proteins of some antigens have immune-toxicity or could cause immune disorders, it is possible to modify them to endow safety and immune protection.

There is no particular limitation to the method to synthesize the modified nucleic acid of the present invention in accordance with an epitope of a wild type target protein. The method is preferable to be in vitro synthesis by PCR. There is no particular limitation to the source of the nucleic acid sequence of the carboxyl terminal moiety comprised by the synthesized nucleic acid of the present invention, but it is preferable to be derived from a portion of pseudomonas exotoxin, and more preferable to be amino acid sequence comprising KDEL or its corresponding sequence.

The method for preparing a target type subunit vaccine of the present invention comprises a subunit vaccine, which is able to induce protection titers and effectively inhibit infection by an Avian Influenza virus. The structure of the protein vaccine comprises: an epitope of; a peptide having the functions of binding and translocation; and a carboxyl terminal KDEL peptide.

In the present invention, the epitope peptide encoding an □ avian flu viral protein encoding is artificially synthesized, instead of isolating and preparing from a natural Avian Influenza virus, and thus obviates the need to contact pathogens that transgress human bodies, so as to improve the safety of the working environment of researchers and to accelerate research speed of vaccines and drugs.

The avain flu virus in the present invention is *orthomyxoviridae* H5N1. The nucleic acid sequences of suitable epitopes of bird flu virus proteins must be modified so that the encoding epitope peptides are identical to those of naturally occurring virus strains, while at the same time achieving high-level expression in desired host cells.

Meanwhile, the synthesized target antigen used in the target type subunit vaccine are preferable to be generated by converting a full-length epitope peptide of a wild type viral protein to nucleotide codons, selecting a portion of the nucleotide sequence that suits the desired functions, or combining several fragments of the nucleotide sequence, and producing the corresponding epitope peptides (synthesized) of the viral protein by microbes.

The synthesized peptides prepared in accordance with the present invention have the effects of inducing antibodies in vivo, while infection during immunization of the subjects is prevented, so they can serve as relative safe vaccines of antibody compositions.

There is no particular limitation to the epitope peptides of avian flu viral proteins suitable to the target type subunit vaccines covered by the present invention but they are preferably selected from one of the group consisting of: H5N1-S1, H5N1-NP, H5N1-HA neutralization titration regions, H5N1-M2, and H5N1-NA enzymatically active sites.

The target subunit vaccines in the present invention, in addition to reacting to antigens, are also functionally related to a vaccine delivery system and have the functions of binding and translocation to antigen presenting cells. There is no particular limitation to the sources of the nucleic acid sequences having the functions of binding and translocation, but they are preferably derived from domain I and domain II of pseudomonas exotoxin. Domain I of pseudomonas exotoxin is a ligand which functions to bind the receptors of a target cell. The suitable target cells can be any one known in the art but they are preferably selected from at least one of the group consisting: T cells, B cells, dendritic cells, monocytes and macrophages. The suitable receptors are selected from at least one of the group consisting of: TGF receptors, IL2 receptors, IL4 receptors, IL6 receptors, 1GF1 receptors, CD4 receptors, IL18 receptors, IL 12 receptors, EGF receptors, LDL receptors, a 2 macroglobulin receptors, and heat shock proteins.

In the target subunit vaccines in the present invention, the target antigen genes are inserted into the homologous loci of the similar genes, and then reverse genetic engineering and eight-plasmid flu system are employed so as to generate a vaccine strain against novel pathogens.

The design idea of the fusion protein in the present invention is to develop vaccines having a conserved common immunogen, e.g. vaccines having antigens like M2. In other words, immune reactions induced from the vaccine comprising the fusion proteins of the present invention can response for various types of influenza viruses, e.g. H5N1, H5N2, H1N1, and so forth, even though the virus mutates vary rapidly. Therefore, the vaccines of the present invention comprising the conserved common immunogen can be used for treating disease infected by the virus without the drawbacks of the conventional vaccines, i.e. changing into or developing new vaccines every year. Hence, this design idea of the vaccines of the present invention is a future trend for the development of an influenza vaccine. The design idea illustrated above is unlike a conventional method, which only focuses on enhancing the protection of neutralizing antibody titer. In fact, treatment for a disease induced from a virus with the conventional vaccine frequently becomes useless if a new mutant virus of the same type virus appears next year. Nevertheless, the vaccine comprising the fusion protein of the present invention can be efficient for various types of viruses even though new viruses appear through mutation quickly.

Other objects, advantages, and novel features of the invention will become more apparent from the following detailed description when taken in conjunction with the accompanying drawings.

### BRIEF DESCRIPTION OF THE DRAWINGS

Fig. 1 is the electrophoresis photo of PCR-sythesized H5N1-NS1;
Fig. 2a-2d is the electrophoresis photo of PCR-sythesized H5N1-NP, wherein 2a refers to the fragment H5N1-NP-a (256bp), 2b refers to the fragment H5N1-NP-b (365bp), 2c refers to the fragment H5N1-NP-c (464bp), and 2d refers to the fragment H5N1-NP-d (2488p);
Fig.3 is the electrophoresis photo of PCR-sythesized H5N1-HA(486bp);
Fig.4 is the electrophoresis photo of PCR-sythesized H5N1-NA(501bp);.
Fig.5 is the vector scheme of H5N1-NS1;
Fig.6 is the vector scheme of H5N1-NP, wherein 6a refers to the fragment H5N1-NP-a, 6b refers to the fragment H5N1-NP-b, 6c refers to the fragment H5N1-NP-c, and 6d refers to the fragment H5N1-NP-d;
Fig.7 is the vector scheme of H5N1-HA;
Fig.8 is the vector scheme of H5N1-eM2;
Fig.9 is the vector scheme of H5N1-NA;
Fig.10 shows the results of protein expression of vector PE-H5N1-NP-a-K3~PE-H5N1-NP-d-K3, wherein the results of a-d are illustrated;
Fig.11 shows the results of protein expression of vector PE-H5N1-HA-K3;
Fig.12 shows the results of protein expression of vector PE-H5N1-eM2-K3;
Fig.13 shows the results of protein expression of vector PE-H5N1-NA-K3;
Fig.14 illustrates the titers of M2 antibody after immunization of mice by different levels of PE-H5N1-eM2-K3 in Example 6

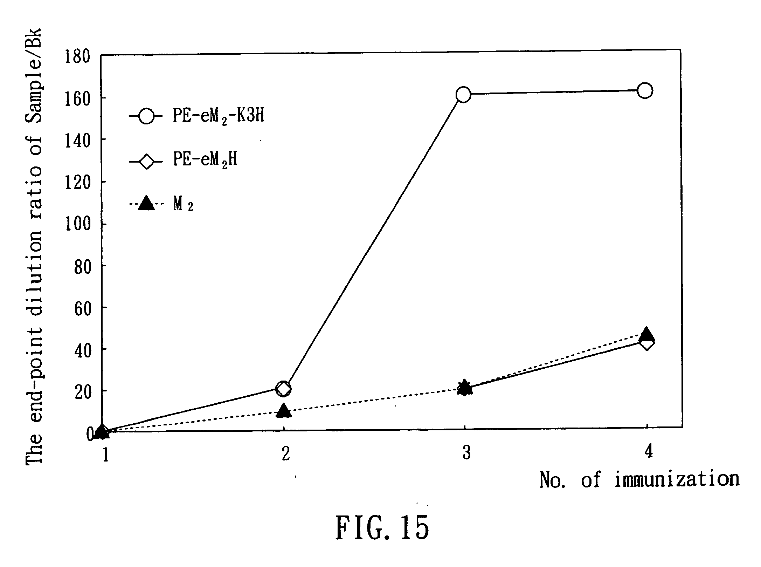

Fig. 15 illustrates changing titers of IgY antibody after immunization of leghorn chicken in Example 7;
Fig.16 shows pathological sections of lungs of the ICR mice after 14 days of challenged with H5N2 type virus in Example 8;
Fig. 17 shows the death rate of chickens during the period of immunization in Example 9;
Fig. 18 shows the egg production of chickens during the period of immunization in Example 9;
Fig.19 illustrates the titer of the antibodies got from the eggs produced by chickens during the period of immunization in Example 9;
Fig.20 illustrates the titer of the 500-times dilution of the antibodies got from the eggs produced by chickens, against H5N1-M2 during the period of immunization in Example 9; and
Fig.21 illustrates the titer of the 500-times dilution of the antibodies got from the eggs produced by chickens, against H5N1-HA during the period of immunization in Example 9.

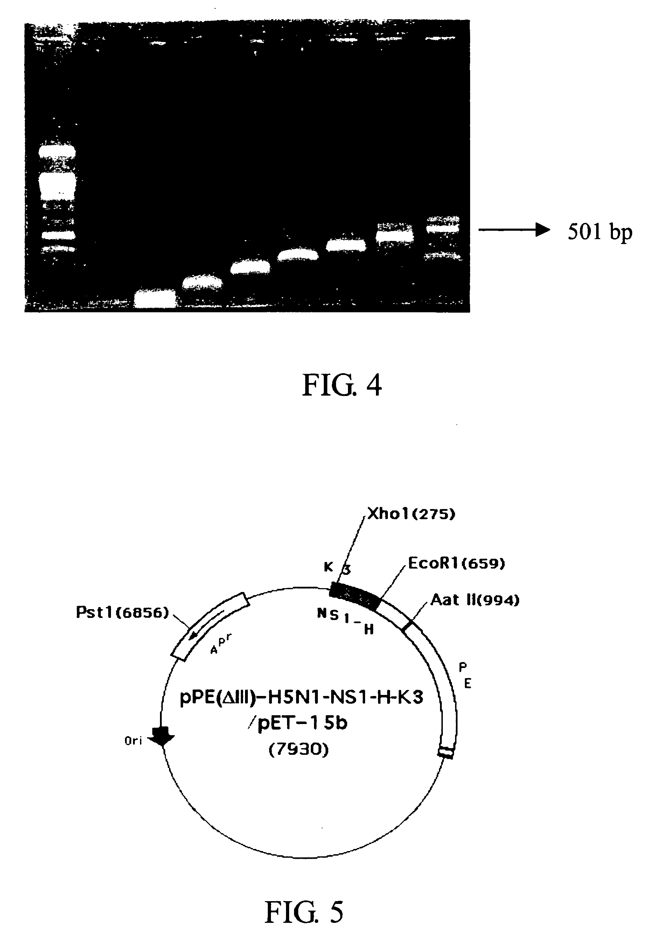

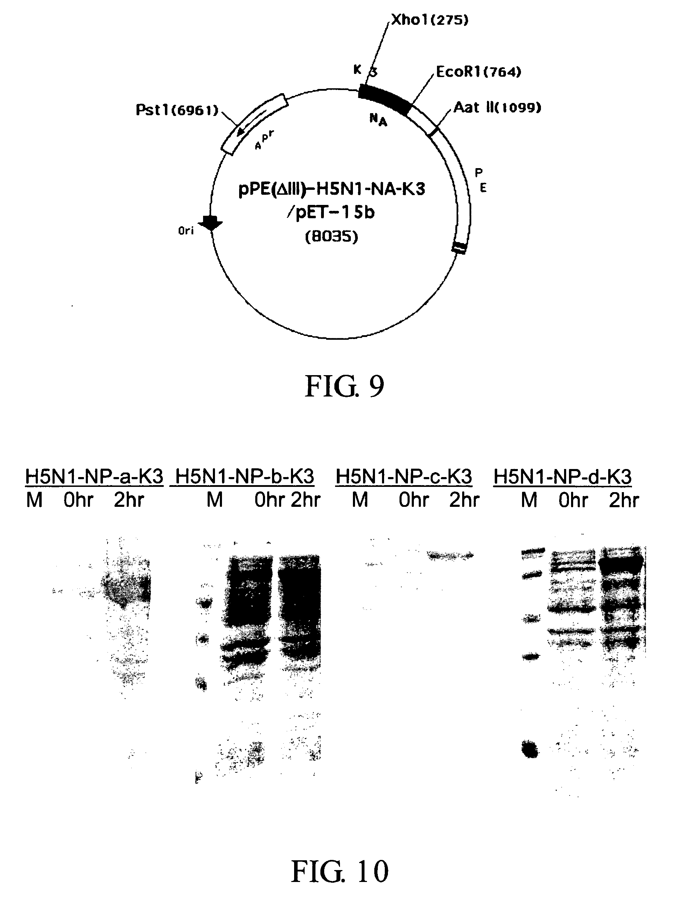

Deposits of the plasmids referred to Figures 5 and 9 have been made at the DSMZ Institute in Germany. Table A below details the deposits made, the accession numbers for the deposits, the date the deposits were made and the Figure numbers the deposits refer to.

**TABLE A**

| **Identification Reference given by the Depositor** | **Accession Number** | **Material** | **Date of the original deposit** | **Figure Number** |
|---|---|---|---|---|
| pPE(deltaIII)-H5N1-NS1-H-K3/pET-15b | DSM 19253 | plasmid | 20070329 | 5 |
| pPE(deltaIII)-NPIA-K3/pET-15b | DSM 19254 | plasmid | 20070329 | 6a |
| pPE(deltaIII)-NPIB-K3/pET-15b | DSM 19255 | plasmid | 20070329 | 6b |
| pPE(deltaIII)-NPIC-K3/pET-15b | DSM 19256 | plasmid | 20070329 | 6c |
| pPE(deltaIII)-NPID-K3/pET-15b | DSM 19257 | plasmid | 20070329 | 6d |
| pPE(deltaIII)-eM2K3/pET-15b | DSM 19258 | plasmid | 20070329 | 8 |
| pPE(deltaIII)-H5N1-NS1-HA-K3/pET-15b | DSM 19259 | plasmid | 20070329 | 7 |
| pPE(deltaIII)-H5N1-NS1-NA-K3/pET-15b | DSM 19260 | plasmid | 20070329 | 9 |

### DETAILED DESCRIPTION OF THE PREFERRED EMBODIMENT

The technical platform for preparing a targeting subunit vaccine of the present invention makes it possible to use a peptide sequence having functions of binding and translocation, and a plasmid of carboxy-terminal KDEL type peptide to construct a plasmid capable of producing a target protein in vitro, after the sequence of the target protein is obtained, codon-converted and modified.

The following embodiments utilize several peptides of avian influenza virus H5N1 as the target antigens.

In accordance with the aforementioned mechanisms of viral infection, the present invention employs highly conserved regions of some key immunogenic proteins among them ( i.e. epitope ) for testing, to elicit immunity in vivo without occurrence of viral infection in the course of research and administration of vaccines. The target proteins used in the following examples are: H5N1-NS1, H5N1-NP, H5N1-HA, H5N1-M2, and H5N1-NA.

The target type subunit plasmid discovered in the experiments shows poor efficiency in the induction of protein synthesis in the host *E. coli* cells, possibly attributable to the toxicity of the protein itself. Therefore, the hydrophobic regions of M2 are removed, and the hydrophilic regions of the protein remain. The modified protein is dubbed HSN1-eM2. Through this modification, HSN1-eM2 could be expressed in large scale in *E.coli.* The encoded amino acids are not influenced and the regions of high immunogenecity are reserved according to the result of the sequence comparison. Thus, the H5N1-M2 related antigens are mainly represented by H5N1-eM2 in the present invention.

### Example 1. The selection of target sequence fragments

The DNA sequences and the correspondent amino acid sequences of H5N1-NS1, H5N1-NP, H5N1-HA, H5N1-M2, and H5N1-NA were retrieved from the National Center of Biotechnology Information (NCBI, USA) database.

According to the theories of vaccine preparation, not all of the peptide sequences of antigen proteins were able to induce the antibodies reactive to a whole antigen protein. The sequence must at least be located on the surface of the antigen and be able to contact water. Thus, the above-mentioned DNA sequences were each entered into the software used for evaluating the hydrophobicity (i.e. DNA strider V1.0), then the desired segments to be synthesized were selected according to the resulting evaluation plot. In this Example, hydrophilic regions were selected to proceed with the preparation of the synthesized peptides. Note that this Example is not set to limit the selection of desired regions, and other regions able to induce similar effects are covered by the claims of the present invention.

According to the above results of evaluation by the software, several hydrophilic segments were selected from each target protein of the Example. The amino acid sequences include: one from H5N1-NS1 ( SEQ. ID. NO. 1 ), four from H5N1-NP( SEQ. ID. NO.2, SEQ. ID. NO.3, SEQ. ID. NO.4, SEQ. ID. NO.5 ), one from H5N1-HA ( SEQ. ID. NO.6 ) , one from H5N1-eM2 SEQ. ID. NO.7), and H5N1-NA ( SEQ. ID. NO.8 ).

The resulting target must undergo restriction digestion, so it was preferable that the target DNA sequences have no restriction site. Thus, software must be employed to evaluate the fact whether these sites reside in the DNA sequences or not. If these sites reside in the DNA sequences, they must be replaced with other condons encoding the same amino acids. The software can also check the existence of the designed restriction sites at both termini of the DNA, which makes following cloning procedures possible.

### Example 2. In vitro synthesis of the target sequences

The nucleic acid sequence encoding the wildtype protein was modified to make the protein be expressed in large scale in *E. coli*; the key point of the modification was to modify each single nucleotide without affecting the originally expressed amino acids, and at the same time to express them effectively in *E. coli.* The nucleic acids of the modified nucleic acid sequence were synthesized by polymerase chain reaction. The primers were numbered as shown in Table 1.

**Table 1**

| Target antigen | Number of forward primers | Seq. ID. No. | Number of forward primers | Seq. ID. No. |
|---|---|---|---|---|
| NS1 | 6 | 9-14 | 6 | 15-20 |
| NP-a | 4 | 21-24 | 4 | 25-28 |
| NP-b | 6 | 29-34 | 6 | 35-40 |
| NP-c | 8 | 41-48 | 8 | 49-56 |
| NP-d | 8 | 57-64 | 8 | 65-72 |
| HA | 8 | 73-80 | 8 | 81-88 |
| eM2 | 4 | 89-92 | 4 | 93-96 |
| NA | 8 | 97-104 | 8 | 105-112 |

First, non-DNA-template PCRs were used by using forward and reverse primers to proceed with enzyme-catalyzed annealing of nucleotide fragments, wherein the 3' ends of each primers have 10-15 bases that were complementary to each other. A PCR DNA product was then generated through reading and complementation of polymerase.

After the first round PCR, 0.01-1µl of the product was used as the DNA template of the second round PCR. The second primer pair was added at a suitable amount together with dNTPs, reagents and Pfu polymerase, and then the second round PCR was performed. Subsequently, different primer pairs were added each time in this manner, so that modified nucleotide sequences were respectively synthesized.

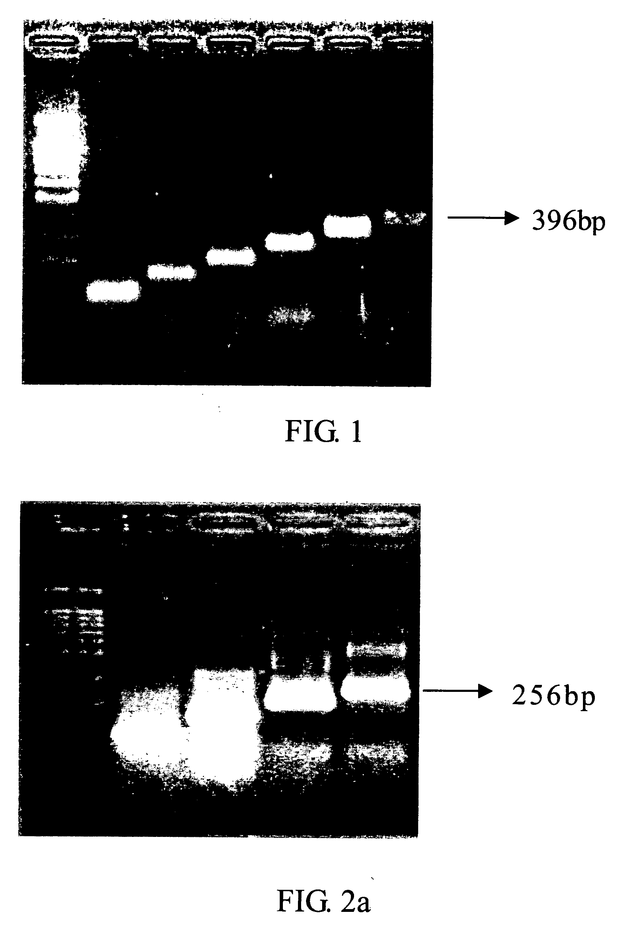

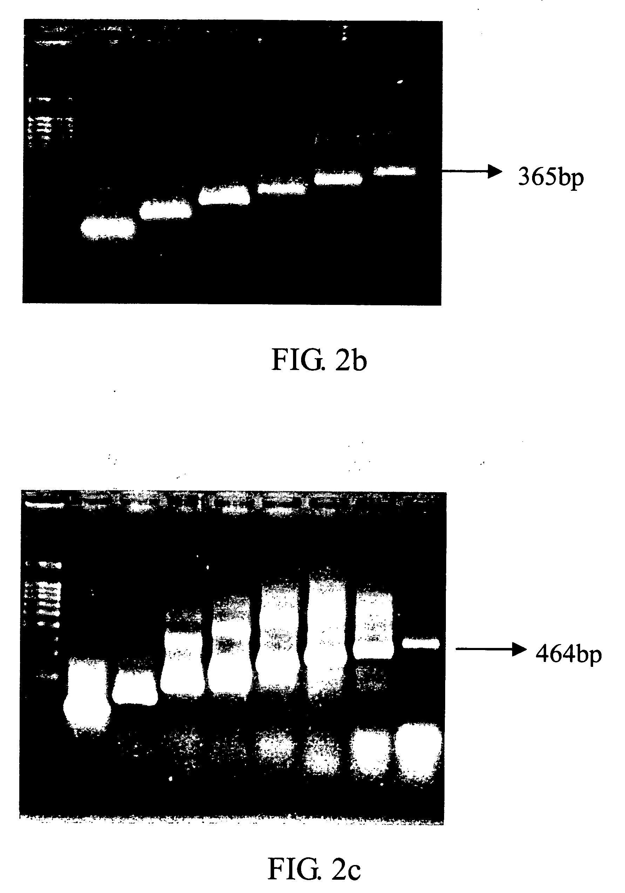

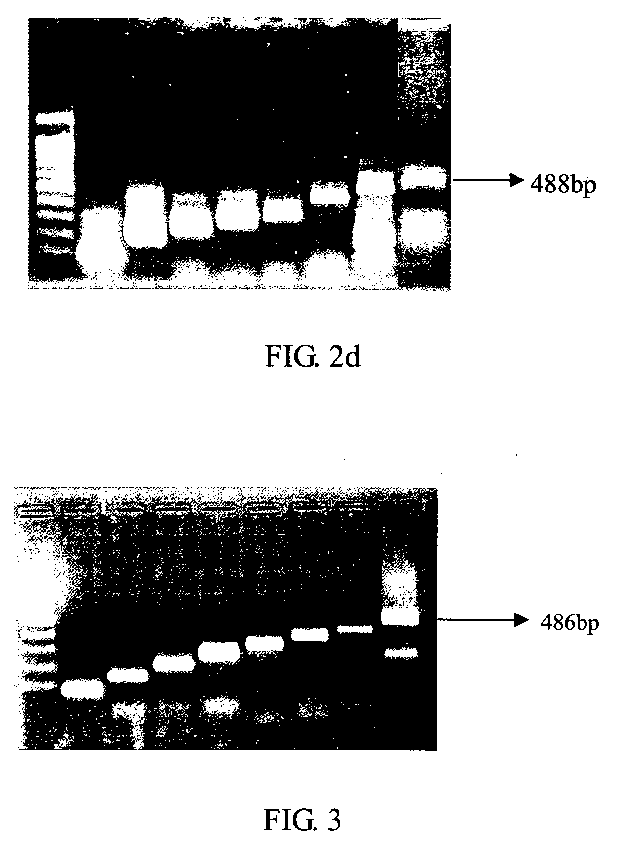

All synthesized nucleotide fragments were analyzed by electrophoresis to check if the produced fragments were of the expected sizes. H5N1-NS (396bp), as shown in Fig. 1; H5N1-NP (four fragments a, b, c and d were used, with 256 bp of fragment a, 365 bp of fragment b, 464 bp of fragment c, and 488bp of fragment d), as shown in Fig. 2a-2d; H5N1-HA (486bp), as shown in Fig.3; H5N1-eM2; and H5N1-HA (501bp), as shown in Fig.4.

### Example 3. Constructing the plasmid comprising the target sequence

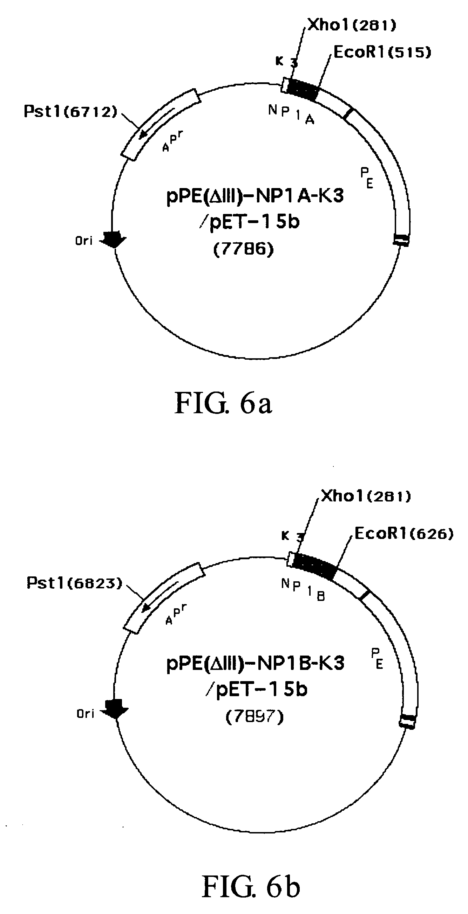

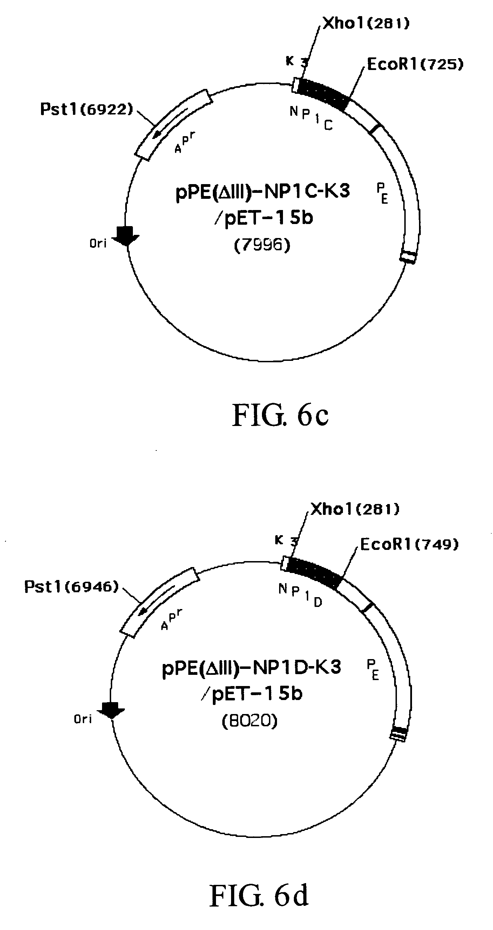

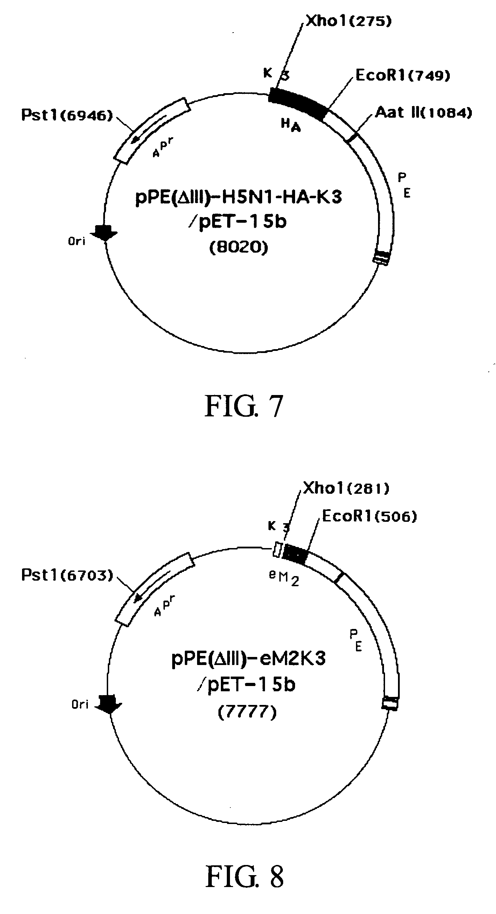

Eight of the aforementioned synthesized fragments (including NP-a~d) were digested by EcoRI and XhoI and then ligated to a peptide sequence having the functions of binding and translocation and an *E.coli* plasmid containing a carboxyl terminal moiety. The plasmids constructed were:
1. pPE-H5N1-NS1-K3 plasmid:
   The construct was built in a pET vector system having an ampicilin resistance fragment, which was able to express H5N1-NS1 fusion protein. (The vector scheme is illustrated in Fig. 5.)
2. pPE-H5N1-NP, a, b, c, d-K3 plasmid:
   This constructed pET15 vector system is able to express H5N1-NP1A~H5N1-NP1D fusion proteins. (The vector scheme is illustrated in Figs. 6a-6d.)
3. pPE-H5N1-HA-K3 plasmid
   This constructed pET15 vector system is able to express H5N1-HA fusion protein. (The vector scheme is illustrated in Fig. 7.)
4. pPE-H5N1-eM2-K3 plasmid
   This constructed pET15 vector system was able to express H5N1-eM2 fusion protein. (The vector scheme is illustrated in Fig. 8.)
5.pPE-H5N1-NA-K3 plasmid
   This constructed pET15 vector system is able to express H5N1-NA fusion protein. (The vector scheme is illustrated in Fig. 9.)

Finally, the above plasmids were transformed into cells of different *E.coli* strains.

### Example 4. Expressing and analyzing the target proteins

The *E.coli* strains, after being checked that 90% of the bacteria populations have the above plasmids with desired genes, were stored at -70 °C in glycerol in 2-ml aliquots.

In a sterile environment, 2 ml of the stored clones was inoculated in a 500ml flask containing 200ml LB(+500µ g /ml Amp), incubated in a 37°C rotating incubator and shaken at 150rpm for 10-12 hours, and the bacteria cultures were generated. OD600 of the cultures should attain 1.0±0.3.

In a sterile environment, 50ml culture liquid was inoculated in each eight sterilized 3000ml flasks, each of them containing 1250ml LB(+500µ g /ml Amp+50ml 10% glucose), incubated in a 37°C rotating incubator and shaken at 150rpm for 2-3 hours, added with IPTG to a final concentration 50ppm, and further incubated in a 37°C rotating incubator and shaken at 150rpm for 2 hours, so that the protein production was completed.

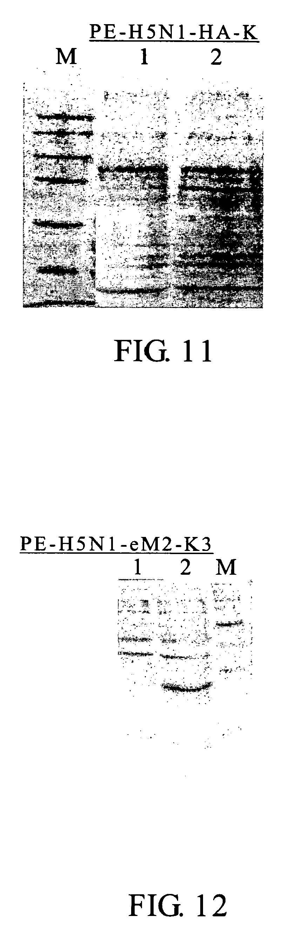

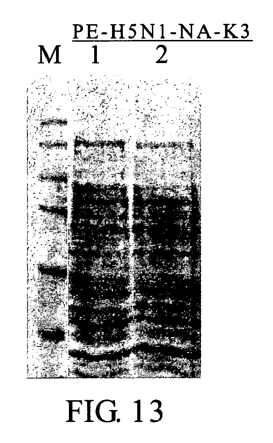

Subsequently, the antigen protein fragments in inclusion bodies were resolved by 8M urea extraction method, such as PE-H5N1-NS1-K3, PE-H5N1-NP-a-K3~PE-H5N1-NP-d-K3(Fig10), PE-H5N1-HA-K3(Fig11), PE-H5N1-eM2-K3(Fig12), and PE-H5N1-NA-K3(Fig13). Each of them was extracted with 10 liter bacteria culture liquid, and 300-400mg antigen was obtained. Totally 3000-9000 injection doses can be obtained from 300 mg antigen.

Each antigen solution was quantified by Western-blotting, coomasie blue staining, and SDS-PAGE electrophoresis with measurement of density of the bands by a densitometer. 0.03±0.003mg of the above antigen protein was used as the primary content of a high-dose injection, and 0.01 ± 0.0001mg was used as the primary content of a low-dose injection.

### Example 5. Preparation of Avian Influenza vaccines

In a class 100 laminar flow, each antigen solution was added with 8M urea to a final volume 40ml, 40 ml was A 206 adjuvant was then added, and the mixture was stirred at 50rpm for 10 minutes in a stirring bucket, sterilized water was added and the stirring speed was increased to 100rpm to further stir for one hour.

The stirring buckets were transferred to a dispensing room for dispensing, capping and labeling with 1ml per dose in each sterilized injection bottle, so that 100 doses of injection of Avian Influenza vaccine were obtained.

### Example 6. The test of immunity of the vaccine using mice as a model organism

Taking PE-H5N1-eM2-K3 as an example, 0.3±0.03mg was used as the primary content of an ultra-high-dose injection(VH), 0.03±0.003mg was used as the primary content of a median dose injection 0.01±0.01mg was used as the primary content of a high-dose injection(H), and 0.01 ± 0.0001mg was used as the primary content of a low-dose injection, which were mixed with adjuvant of different doses (Spec was A 206) and used to immunize Balc/C mice, each group having 12 mice; the mice were immunized in two weeks and had to be immunized 3 to 4 times in total.

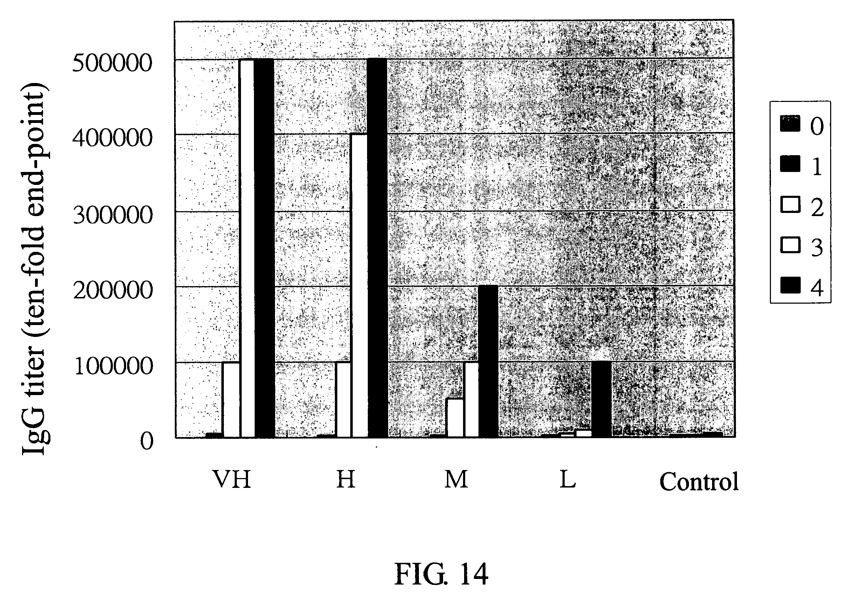

A blood sample was taken after immunization and assayed by ELISA. For M2 anti-specific IgG, an antibody titer was detected after the second round of immunization by anti-eM2 ELIZA through 1/10 dilution end-point titration assay. Ultra-high-dose injection(VH)(0.1±0.01mg)and High-dose injection(H)(0.3±0.03mg) induced similar titers; the titers peaked after the third round immunization and reached a plateau after the fourth round. The median dose of injection and the low dose of injection induce lower titers, but both reached 10,0000 times after the fourth round immunization. The results are shown in Fig. 14.

### Example 7. Vaccinal immunization tests of Egg-laying Leghorn chicken model

Taking PE-H5N1-eM2-K3 as an example, 0.1±0.01mg of the fusion antigen was used as a dose of injection, mixed with appropriated adjuvants, and administered to a Leghorn chicken at egg-laying stage. After three to four times of immunization, high-titer anti-avian influenza antibodies were accumulated in the yolks, with the titer of ELIZA 1/10 dilution end-point titration assay higher than 10,0000. However, when the antigens used were PE-H5N1-eM2 and or eM2 subunit protein antigen, the IgY titers were very low, only 10-100 times as high as those of the blank, non-immunized group.

### Example 8. Immunization and virus challenge test of AI subunit vaccines in ICR mice

The fusion proteins expressed in the present invention are conserved common immunogens of the H5N1 type influenza virus. According to the knowledge of the influenza virus, a person having the ordinary skill in the art understands that H1N1 type virus has N1 type characteristics of H5N1 type virus, and H5N2 type virus has H5 type characteristics of H5N1 type virus. Hence, vaccines comprising the fusion proteins, which are conserved common immunogens of the H5N1 type influenza virus, expressed in the present invention can protect a host against both of H1N1 type and H5N2 type virus. However, because H5N1 type influenza virus is extremely harmful to human bodies, it is unsuitable to perform experiments by using this type virus directly. Through being challenged respectively with H1N1 type and H5N2 type influenza virus, the vaccines of the present invention can be proved that they are really efficient to H5N 1 type virus due to the H1N1 type and H5N2 type virus respectively having N1 type and H5 type characteristics of H5N1 type virus.

### A. Challenged with H1N1 type human influenza virus

First, ICR mice are separated into five groups, and each group possesses six ICR mice. Each of the different fusion proteins is taken with a determined dose of injection, and then is mixed with an appropriate dose of an adjuvant. They are as follows: ICR mice of Group I are immunized with PE-H5N1-eM2-K3 (H, 0.1±0.01 mg); ICR mice of Group II are immunized with PE-H5N1-NP-(a+b+c+d)-K3 (H, 0.1±0.01 mg); ICR mice of Group III are immunized with PE-H5N1-HA-K3 (H, 0.1±0.01 mg); ICR mice of Group IV are immunized with PE-H5N1-NS1-K3 (L, 0.01±0.001 mg); and ICR mice of Group V are a blank group immunized with nothing.

After three to four times of immunization, the immunized ICR mice of Groups I~V are challenged with H1N1 virus. After four days of post-challenged, the saliva of each ICR mouse in each group is tested for checking the existence of virus excretion. Besides, the healthy condition of every mouse in each group is also observed and recorded. The results are shown in Table 2.

**Table 2**

| Group | Fusion protein | number of immunized mice | number of mice excreting virus at 4 Dpc* | number of ill mice at 4 Dpc* |
|---|---|---|---|---|
| I | PE-H5N1-eM2-K3 (H) | 6 | 2 | 1 |
| II | PE-H5N1-NP-(a+b+c+d)-K3 (H) | 6 | 2 | 0 |
| III | PE-H5N1-HA-K3 (H) | 6 | 5 | 3 |
| IV | PE-H5N1-NS1-K3 (L) | 6 | 4 | 4 |
| Blank group | --- | 6 | 5 | 5 |

| | | | | |
|---|---|---|---|---|
| * Dpc: days of post-challenged. | | | | |

According to Table 2, after four days of post-challenged, the ICR mice immunized with the fusion proteins of the present invention have fewer mice with virus excretion in saliva than those of the blank group. Furthermore, the PE-H5N1-eM2-K3 and PE-H5N1-NP-(a+b+c+d)-K3 fusion proteins in the high dose exhibit the best vaccinal effect, and they can decrease the number of mice with virus excretion in saliva. Moreover, although other fusion proteins do not exhibit the same effect of PE-H5N1-eM2-K3 and PE-H5N1-NP-(a+b+c+d)-K3, they still have better effect against avian influenza than the blank group does.

### B. Challenged with H1N1 type human influenza virus

First, ICR mice are separated into six groups, and each group possesses five ICR mice. Each of the different fusion proteins is taken with a determined dose of injection, and then is mixed with an appropriate dose of an adjuvant. They are as follows: ICR mice of Group I are immunized with PE-H5N1-eM2-K3 (H, 0.1±0.01 1 m g); ICR mice of Group II are immunized with PE-H5N1-NP-(a+b+c+d)-K3 (H, 0.1±0.01 mg); ICR mice of Group III are immunized with PE-H5N1-HA-K3 (L, 0.01±0.001 mg); ICR mice of Group IV are immunized with PE-H5N1-NS1-K3 (L, 0.01 ± 0.001 mg); ICR mice of Group V are immunized with PE-H5N1-NA-K3 (H, 0.1±0.01 mg); and ICR mice of Group VI are a blank group immunized with nothing.

After three to four times of immunization, the immunized ICR mice of Groups I~VI are challenged with H1N1 virus. After four days of post-challenged, the salvia of each ICR mouse in each group is tested for checking the existence of virus excretion. Besides, the healthy condition of every mouse in each group is also observed and recorded. The results are shown in Table 3.

**Table 3**

| Group | Fusion protein | number of immunized mice | number of mice excreting virus at 4 Dpc* | number of ill mice at 4 Dpc* |
|---|---|---|---|---|
| I | PE-H5N1-eM2-K3 (H) | 5 | 2 | 1 |
| II | PE-H5N1-NP-(a+b+c+d)-K3 (H) | 5 | 2 | 0 |
| III | PE-H5N1-HA-K3 (H) | 5 | 2 | 0 |
| IV | PE-H5N1-NS1-K3 (L) | 5 | 2 | 0 |
| V | PE-H5N1-NA-K3 (H) | 5 | 2 | 0 |
| Blank group | --- | 5 | 4 | 2 |

| | | | | |
|---|---|---|---|---|
| * Dpc: days of post-challenged. | | | | |

According to Table 3, after four days of post-challenged, the ICR mice immunized with the fusion proteins of the present invention have fewer mice with virus excretion in saliva than those of the blank group do. Further, all of the fusion proteins in the present invention have better effect against avian influenza than the blank group does.

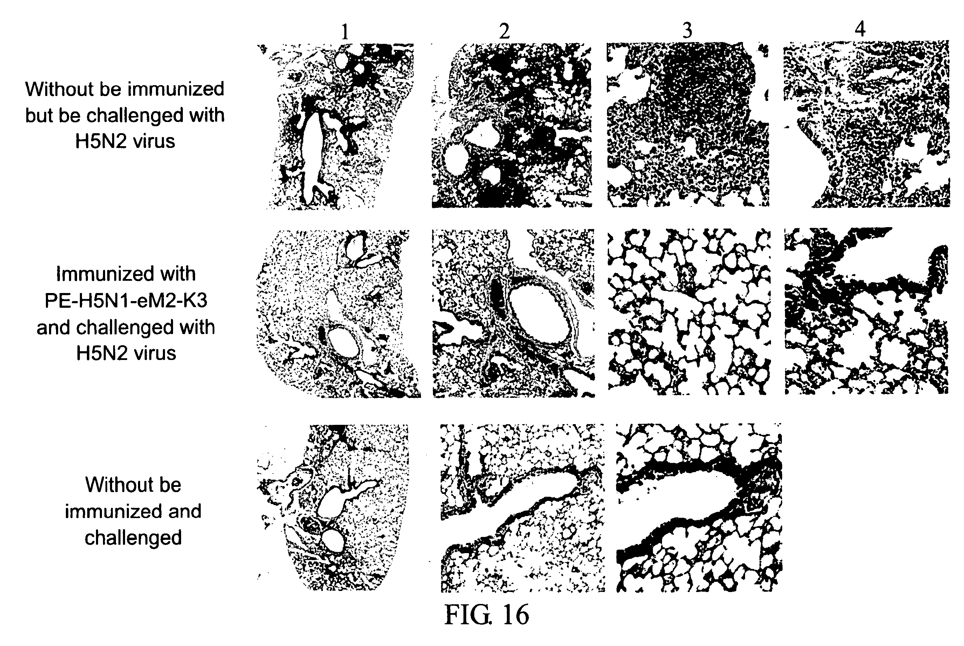

Additionally, after 14 days of post-challenged, ICR mice immunized with PE-H5N1-eM2-K3 are anatomized and their lungs are taken to process pathological sections. As compared with the pathological lung sections of the unimmunized and unchallenged ICR mice and unimmunized and challenged ICR mice, severity levels of interstitial pneumonia are determined and recorded. The results are shown in Table 4 and FIG. 16.

**Table 4**

| group | mice No. | total scores |
|---|---|---|
| unimmunized and challenged ICR mice | BK-1 | 7 |
| | BK-2 | 6 |
| | BK-3 | 7 |
| | BK-4 | 5 |
| ICR mice immunized with PE-H5N1-eM2-K3 and challenged with H5N2 type virus | 8-1 | 2 |
| | 8-2 | 3 |
| | 8-3 | 3 |
| | 8-4 | 4 |
| unimmunized and unchallenged ICR mice | CTL-1 | 1 |
| | CTL-2 | 1 |
| | CTL-3 | 1 |
| | CTL -4 | 1 |

| | | |
|---|---|---|
| *Score marker: minimal=1, mild=2, moderate=3, and severe=4; in addition, multifocal=1, diffuse=2, and subacute=1; the most severe mouse is scored as 7 points; and the mouse with little interstitial pneumonia is scored as 3 points. | | |

With reference to FIG. 16 and Table 4, compared with the unimmunized and challenged ICR mice, the ICR mice immunized with PE-H5N1-eM2-K3 have few symptoms of interstitial pneumonia close to the ICR mice without being challenged with H5N2 type virus.

### Example 9. Field trial in a chicken farm infected with H5N2 type avian influenza virus

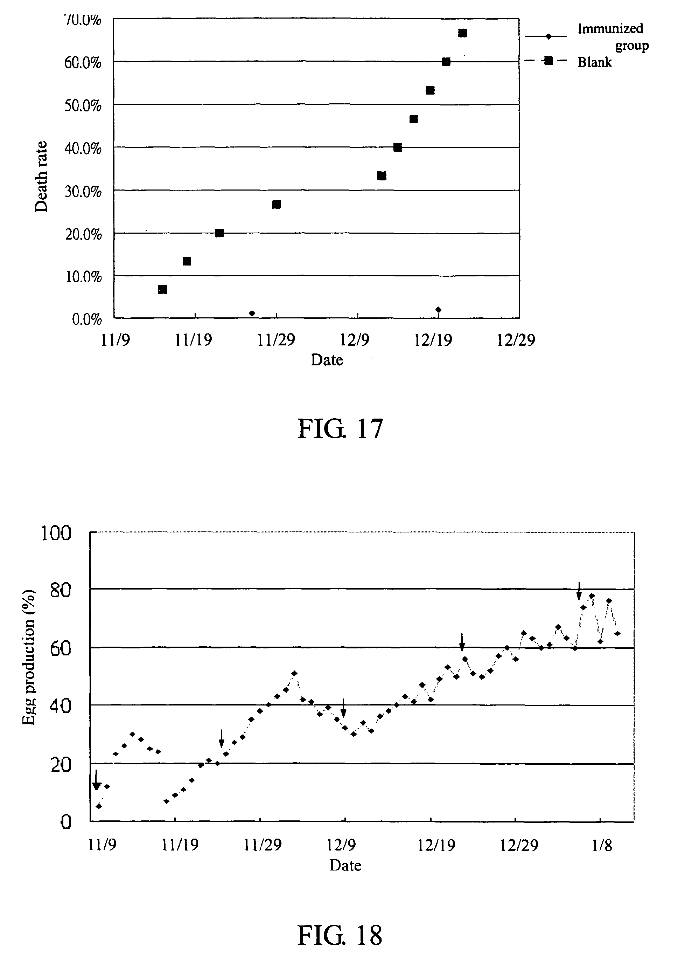

A field trial in a chicken farm broken out with H5N2 type avian influenza virus is performed through immunizing the chickens in the chicken farm with a dose of a complex vaccine comprising 0.05 mg PE-H5N1-eM2-K3, 0.01 mg PE-H5N1-NP-a-K3, 0.01 mg PE-H5N1-NP-b-K3, 0.01 mg PE-H5N1-NP-c-K3, 0.01 mg PE-H5N1-NP-d-K3, 0.05 mg PE-H5N1-HA-K3, 0.05 mg PE-H5N1-NA-K3, and ISA206 of 10%. The complex vaccine is prepared by the way illustrated in Example 9. Additionally, the immunized chickens are immunized again every two weeks till four or five times of immunization are achieved. By comparing the immunized chickens and the chickens in a blank group without being immunized by the above complex vaccine, as shown in FIG. 17, the death rate of the chickens immunized with the above complex vaccine is decreased to under about 5%. However, the death rate of the chickens in the black group without being immunized by the above complex vaccine is raised to about 60% to 70% by time pass.

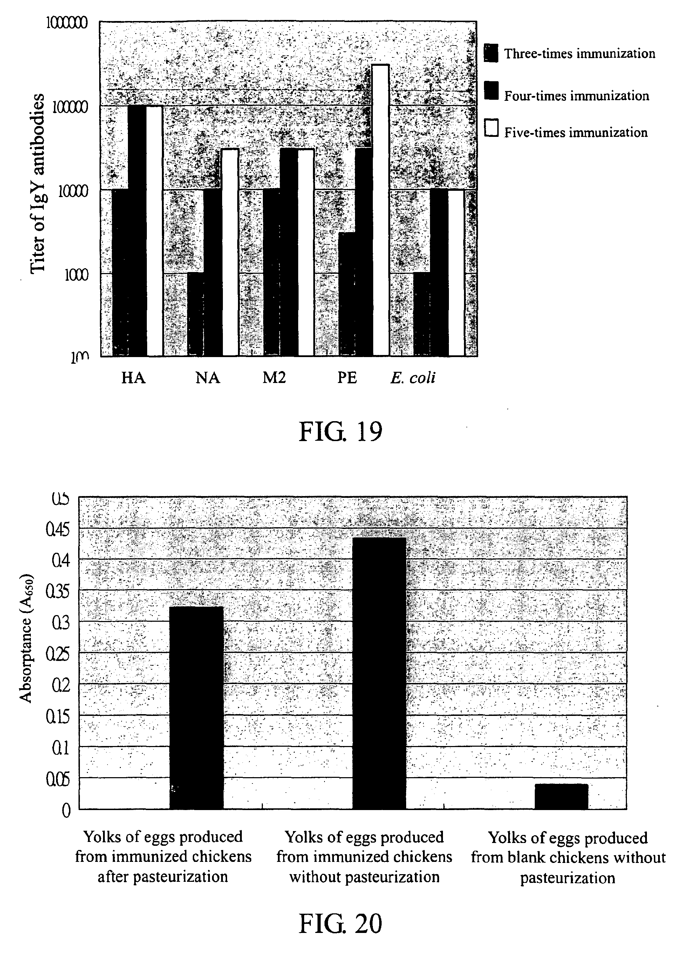

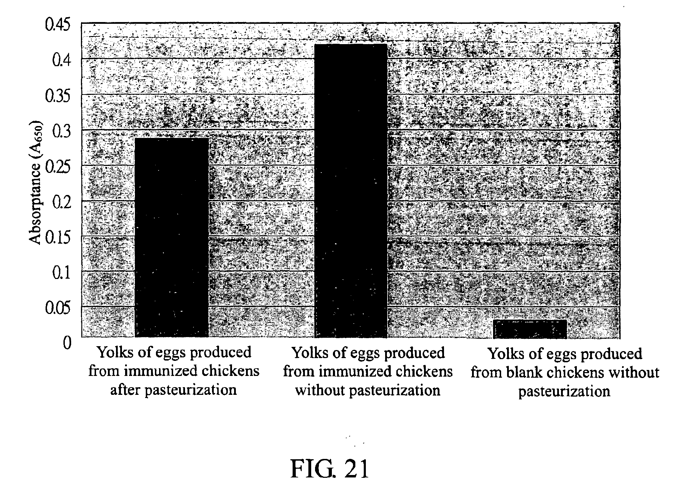

In addition, the egg production of the immunized chickens tends upwards by times of immunization, and the results are shown in FIG 18. The yolks got from eggs produced by the immunized chickens after three to five times of immunization perform ten-fold serial end-point diffusion test to check the titer of IgY antibodies. As shown in FIG 19, the titer of the IgY antibodies against HA, NA, M2, PE, or *E*. *coli* is increased by the times of immunization. Furthermore, the yolks got from eggs produced by the immunized chickens after five times of immunization are also tested to check the titer of IgY antibodies against H5N1-M2 or H5N1-HA. We discover that 500 times dilution of the antibodies still has dramatic positive reaction, and the results are shown in FIG. 20 and FIG 21 respectively.

In conclusion, the method of vaccine preparation obviates the needs to contact highly hazardous biological samples and viral materials. Instead, the amino acid sequence is retrieved directly from the Internet, enabling researchers to generate a safe and effective vaccine. The establishment of this platform is essential for vaccinal preparation in countries that are currently unaffected by but highly vulnerable to the infectious disease. The design idea of the fusion protein in the present invention is to develop a conserved common immunogen, e.g. vaccines having antigens like M2. In other words, immune reactions induced from the vaccine comprising the fusion proteins of the present invention can response for various types of influenza viruses, e.g. H5N1, H5N2, H1N1, and so forth, even though the virus mutates vary rapidly. Therefore, the vaccines of the present invention comprising the conserved common immunogen can be used for treating disease infected by the virus without the drawbacks of the conventional vaccines, i.e. changing into or developing new vaccines every year. Hence, this design idea of the vaccines of the present invention is a future trend for the development of an influenza vaccine. The design idea illustrated above is unlike a conventional method, which only focuses on enhancing the protection of neutralizing antibody titer. In fact, treatment for new mutant virus with the conventional vaccine frequently becomes useless if a new mutant virus of the same type virus appears next year. Nevertheless, the vaccine comprising the fusion protein of the present invention can be efficient for various type viruses even though new viruses appear through mutation quickly.

Although the present invention has been explained in relation to its preferred embodiment, it is to be understood that many other possible modifications and variations can be made without departing from the scope of the invention as hereinafter claimed.

## Claims

1. A method for preparing a protein vaccine or a vaccinal virus strain, comprising:
( a ) providing an amino acid sequence of at least one epitope peptide of a target antigen protein, and converting the amino acid sequence to a corresponding wildtype nucleic acid sequence;
( b ) modifying the wildtype nucleic acid sequence of the epitope to a modified nucleic acid sequence which is recognizable to a host cell and encodes the epitope peptide;
( c ) synthesizing primers of the modified nucleic acid sequence, wherein the primers are nucleic sequences having 5-200 nucleic acids, the primers are identical or complementary to portions of the modified nucleic acid sequence, and the 3' ends of the forward primers and the 3' ends of the reverse primers among the primers comprise sequences of 5-20 nucleic acids that are complementary to each other;
( d ) synthesizing the modified nucleic acid in vitro using the primers;
( e ) linking the synthesized nucleic acid fragments to a nucleic acid sequence having the functions of binding and translocation and a plasmid having carboxyl terminal moiety to produce a modified plasmid;
( f ) transforming the modified plasmid into a host cell, so as to produce the epitope peptide encoded by the modified nucleic acid; and
( g ) collecting and purifying the epitope peptide.

2. The method of claim 1, wherein the amino acid sequence of the epitope peptide is not naturally derived.

3. The method of claim 1 or claim 2, wherein the epitope peptide is a hydrophilic region on the structure of the antigen protein.

4. The method of claim 1 or claim 2, wherein the epitope peptide is a hydrophobic region on the structure of the antigen protein.

5. The method of any preceding claim, wherein the host cell is a microbial cell, a plant cell or an animal cell.

6. The method of any preceding claim, wherein the host cell is an *E.coli* cell or a yeast cell.

7. The method of any preceding claim, wherein the wildtype nucleic acid sequence encodes the same peptide as the synthesized nucleic acid sequence does.

8. The method of any preceding claim, wherein the synthesized nucleic acid sequence is synthesized by polymerase chain reaction.

9. The method of any preceding claim, wherein the nucleic acid sequence having the functions of binding and translocation is derived from the domain I and domain II of pseudomonas exotoxin.

10. The method of any preceding claim, wherein the target antigen genes are inserted into the homologous loci of the similar genes in the vaccine strain, and then reverse genetic engineering and eight-plasmid flu system are employed so as to generate a vaccine strain against novel pathogens.

11. The method of claim 9, wherein the pseudomonas exotoxin is a ligand.

12. The method of claim 11, wherein the ligand binds a receptor of the host cell.

13. The method of claim 12, wherein the host cell is selected from a group consisting of: T cells, B cells, dendritic cells, monocytes and macrophages.

14. The method of claim 12, wherein the receptor is selected from a group consisting of: TGF receptors, IL2 receptors, IL4 receptors, IL6 receptors, 1GF1 receptors, CD4 receptors, IL 18 receptors, IL12 receptors, EGF receptors, LDL receptors, α 2 macroglobulin receptors, and heat shock proteins.

15. The method of any preceding claim, wherein the carboxyl terminal moiety is derived from a portion of the pseudomonas exotoxin.

16. The method of any preceding claim, wherein the carboxyl terminal moiety comprises a KDEL amino acid sequence and the corresponding nucleic acid sequence.

17. A protein vaccine for inhibiting an Avian Influenza virus, wherein the structure of the protein vaccine comprising: an epitope peptide of the Avian Influenza virus; a peptide having the functions of binding and translocation; and a carboxyl terminal moiety.

18. The protein of claim 17, wherein the epitope peptide encoding the Avian Influenza virus protein is artificially synthesized.

19. The protein of claim 17 or 18, wherein the Avian Influenza virus is *orthomyxoviridae* H5N1.

20. The protein of claim 17, 18 or 19, wherein the epitope peptide of the Avian Influenza virus is modified.

21. The protein of claim 17, 18 , 19 or 20, wherein the epitope peptide of the Avian Influenza virus is selected from a group consisting: H5N1-NS1 , H5N1-NP , H5N1-HA , H5N1-eM2 , and H5N1-NA.

22. The protein of any of claims 17 to 21, wherein the peptide having the functions of binding and translocation is derived from the domain I and domain II of pseudomonas exotoxin.

23. The protein of claim 22, wherein the pseudomonas exotoxin is a ligand.

24. The protein of claim 23, wherein the ligand binds a receptor of the host cell.

25. The protein of claim 24, wherein the host cell is selected from a group consisting of: T cells, B cells, dendritic cells, monocytes and macrophages.

26. The protein of claim 24, wherein the receptor is selected from a group consisting of: TGF receptors, IL2 receptors, IL4 receptors, IL6 receptors, 1GF1 receptors, CD4 receptors, IL 18 receptors, IL 12 receptors, EGF receptors, LDL receptors, α 2 macroglobulin receptors, and heat shock proteins.

27. The protein of any of claims 17 to 26, wherein the carboxyl terminal moiety is derived from a portion of the pseudomonas exotoxin.

28. The protein of any of claims 17 to 27, wherein the carboxyl terminal moiety comprises a KDEL amino acid sequence.


SEQUENCE LISTING

<110>  HealthBanks Biotech Co. Ltd.

<120>  Using a Reverse Genetic Engineering Platform to Produce Vaccines
       or Functional Proteins

<130>  ADT30092P.EPP

<150>  US 60/791,880
<151>  2006-04-14

<150>  US - number not yet known
<151>  2007-04-09

<160>  112

<170>  PatentIn version 3.3

<210>  1
<211>  120
<212>  PRT
<213>  Avian influenza virus

<400>  1

Met Asp Ser Asn Thr Val Ser Ser Phe Gln Val Asp Cys Phe Leu Trp
1               5                   10                  15

Arg Val Arg Lys Arg Phe Ala Asp Gln Glu Leu Gly Asp Ala Pro Phe
            20                  25                  30

Leu Asp Arg Leu Arg Arg Asp Gln Lys Ser Leu Arg Gly Arg Gly Ser
        35                  40                  45

Thr Leu Gly Leu Asp Ile Arg Thr Ala Thr Arg Glu Gly Lys His Ile
    50                  55                  60

Val Glu Arg Ile Leu Glu Glu Glu Ser Asp Glu Ala Leu Lys Met Thr
65                  70                  75                  80

Ile Ala Ser Val Pro Ala Pro Arg Tyr Leu Thr Glu Met Thr Leu Glu
                85                  90                  95

Glu Met Ser Arg Asp Trp Leu Met Leu Ile Pro Lys Gln Lys Val Thr
            100                 105                 110

Gly Ser Leu Cys Ile Arg Met Asp
        115                 120

<210>  2
<211>  70
<212>  PRT
<213>  Avian influenza virus

<400>  2

Met Ala Ser Gln Gly Thr Lys Arg Ser Tyr Glu Gln Met Glu Thr Gly
1               5                   10                  15

Gly Glu Arg Gln Asn Ala Thr Glu Ile Arg Ala Ser Val Gly Arg Met
            20                  25                  30

Ile Ser Gly Ile Gly Arg Phe Tyr Ile Gln Met Cys Thr Glu Leu Lys
        35                  40                  45

Leu Ser Asp Tyr Glu Gly Arg Leu Ile Gln Asn Ser Lys Lys Asp Glu
    50                  55                  60

Leu Arg Asp Glu Leu Lys
65                  70

<210>  3
<211>  107
<212>  PRT
<213>  Avian influenza virus

<400>  3

Arg Met Val Leu Ser Ala Phe Asp Glu Arg Arg Asn Arg Tyr Leu Glu
1               5                   10                  15

Glu His Pro Ser Ala Gly Lys Asp Pro Lys Lys Thr Gly Gly Pro Ile
            20                  25                  30

Tyr Arg Arg Arg Asp Gly Lys Trp Val Arg Glu Leu Ile Leu Tyr Asp
        35                  40                  45

Lys Glu Glu Ile Arg Arg Ile Trp Arg Gln Ala Asn Asn Gly Glu Asp
    50                  55                  60

Ala Thr Ala Gly Leu Thr His Leu Met Ile Trp His Ser Asn Leu Asn
65                  70                  75                  80

Asp Ala Thr Tyr Gln Arg Thr Arg Ala Leu Val Arg Thr Gly Met Asp
                85                  90                  95

Pro Arg Met Cys Ser Lys Lys Asp Glu Leu Lys
            100                 105

<210>  4
<211>  140
<212>  PRT
<213>  Avian influenza virus

<400>  4

Thr Leu Pro Arg Arg Ser Gly Ala Ala Gly Ala Ala Val Lys Gly Val
1               5                   10                  15

Gly Thr Met Val Met Glu Leu Ile Arg Met Ile Lys Arg Gly Ile Asp
            20                  25                  30

Asp Arg Asn Phe Trp Arg Gly Glu Asn Gly Arg Arg Thr Arg Ile Ala

35                  40                  45

Tyr Glu Arg Met Cys Asn Ile Leu Lys Gly Lys Phe Gln Thr Ala Ala
    50                  55                  60

Gln Arg Ala Met Met Asp Lys Lys Asp Glu Leu Leu Ile Phe Leu Ala
65                  70                  75                  80

Arg Ser Ala Leu Ile Leu Arg Gly Ser Val Ala His Lys Ser Cys Leu
                85                  90                  95

Pro Ala Cys Val Tyr Gly Leu Ala Val Ala Ser Gly Tyr Asp Phe Glu
            100                 105                 110

Arg Glu Gly Tyr Ser Leu Val Gly Ile Asp Pro Phe Arg Leu Leu Gln
        115                 120                 125

Asn Ser Gln Val Phe Ser Leu Arg Asp Glu Leu Lys
    130                 135                 140

<210>  5
<211>  148
<212>  PRT
<213>  Avian influenza virus

<400>  5

Phe Ile Arg Gly Thr Arg Val Val Pro Arg Gly Gln Leu Ser Thr Arg
1               5                   10                  15

Gly Val Gln Ile Ala Ser Asn Glu Asn Met Glu Ala Met Asp Ser Asn
            20                  25                  30

Thr Leu Glu Leu Arg Ser Arg Tyr Trp Ala Ile Arg Thr Arg Ser Gly
        35                  40                  45

Gly Asn Leu Glu Asp Lys Lys Thr Phe Ser Val Gln Arg Asn Leu Pro
    50                  55                  60

Phe Glu Arg Ala Thr Ile Met Ala Val Phe Thr Gly Asn Thr Glu Gly
65                  70                  75                  80

Arg Thr Ser Asp Met Arg Thr Glu Ile Ile Arg Met Met Glu Ser Ala
                85                  90                  95

Arg Pro Glu Asp Val Ser Phe Gln Gly Arg Gly Val Phe Glu Leu Ser
            100                 105                 110

Asp Glu Lys Ala Thr Asn Pro Ile Val Pro Ser Phe Asp Met Asn Asn
        115                 120                 125

Glu Gly Ser Tyr Phe Phe Gly Asp Asn Ala Glu Glu Tyr Tyr Asn Arg
    130                 135                 140

Asp Glu Leu Lys
145

<210> 6
<211> 150
<212> PRT
<213> Avian influenza virus

<400> 6

Ala Asn Asp Leu Cys Tyr Pro Gly Asp Phe Asn Asp Tyr Glu Glu Leu
1               5                   10                  15

Lys His Leu Leu Ser Arg Ile Asn His Phe Glu Lys Ile Gln Ile Ile
            20                  25                  30

Pro Lys Ser Ser Trp Ser Asn His Glu Ala Ser Ser Gly Val Ser Ser
        35                  40                  45

Ala Cys Pro Tyr Leu Gly Lys Ser Ser Phe Phe Arg Asn Val Val Trp
    50                  55                  60

Leu Ile Lys Lys Asn Asn Ala Tyr Pro Thr Ile Lys Arg Ser Tyr Asn
65                  70                  75                  80

Asn Thr Asn Gln Glu Asp Leu Leu Val Leu Trp Gly Ile His His Pro
                85                  90                  95

Asn Asp Ala Ala Glu Gln Thr Lys Leu Tyr Gln Asn Pro Thr Thr Tyr
            100                 105                 110

Ile Ser Val Gly Thr Ser Thr Leu Asn Gln Arg Leu Val Pro Lys Ile
        115                 120                 125

Ala Thr Arg Ser Lys Val Asn Gly Gln Ser Gly Arg Met Glu Phe Phe
    130                 135                 140

Trp Thr Ile Leu Lys Pro
145                 150

<210> 7
<211> 70
<212> PRT
<213> Avian influenza virus

<400> 7

Met Ser Leu Leu Thr Glu Val Glu Thr Pro Thr Arg Asn Glu Trp Glu
1               5                   10                  15

Cys Arg Cys Ser Asp Ser Ser Asp Pro Ile Tyr Arg Arg Leu Lys Tyr
            20                  25                  30

Gly Leu Lys Arg Gly Pro Ser Thr Ala Gly Val Pro Glu Ser Met Arg
        35                  40                  45

Glu Glu Tyr Arg Gln Glu Gln Gln Ser Ala Val Asp Val Asp Asp Gly
    50                  55                  60

His Phe Val Asn Ile Glu
65                  70

<210> 8
<211> 155
<212> PRT
<213> Avian influenza virus

<400> 8

Gly Tyr Ile Cys Ser Gly Val Phe Gly Asp Asn Pro Arg Pro Asn Asp
1               5                   10                  15

Gly Thr Gly Ser Cys Gly Pro Met Ser Leu Asn Gly Ala Tyr Gly Val
            20                  25                  30

Lys Gly Phe Ser Phe Lys Tyr Gly Asn Gly Val Trp Ile Gly Arg Thr
        35                  40                  45

Lys Ser Thr Asn Ser Arg Ser Gly Phe Glu Met Ile Trp Asp Pro Asn
    50                  55                  60

Gly Trp Thr Gly Thr Asp Ser Ser Phe Ser Val Lys Gln Asp Ile Val
65                  70                  75                  80

Ala Ile Thr Asp Trp Ser Gly Tyr Ser Gly Ser Phe Val Gln His Pro
                85                  90                  95

Glu Leu Thr Gly Leu Asp Cys Ile Arg Pro Cys Phe Trp Ile Glu Leu
            100                 105                 110

Ile Arg Gly Arg Pro Lys Glu Ser Thr Ile Trp Thr Ser Gly Ser Ser
        115                 120                 125

Ile Ser Phe Cys Gly Val Asn Ser Asp Thr Val Gly Trp Ser Trp Pro
    130                 135                 140

Asp Gly Ala Glu Leu Pro Phe Thr Ile Asp Lys
145                 150                 155

<210> 9
<211> 45
<212> DNA
<213> Artificial

<220>
<223> An artificial primer of NS1.

<400> 9

ggttccaccc tgggtctgga catccgtacc gctacccgtg aaggt 45

<210> 10
<211> 45
<212> DNA
<213> Artificial

<220>
<223> An artificial primer of NS1.

<400> 10
cgtcgtgacc agaaatccct gcgtggtcgt ggttccaccc tgggt 45

<210> 11
<211> 49
<212> DNA
<213> Artificial

<220>
<223> An artificial primer of NS1.

<400> 11
gaactgggtg acgctccgtt cctggaccgt ctgcgtcgtg accagaaaa 49

<210> 12
<211> 48
<212> DNA
<213> Artificial

<220>
<223> An artificail primer of NS1.

<400> 12
ctgtggcgtg ttcgtaaacg tttcgctgac caggaactgg gtgacgct 48

<210> 13
<211> 46
<212> DNA
<213> Artificial

<220>
<223> An artificial primer of NS1.

<400> 13
caccgtttcc tccttccagg ttgactgctt cctgtggcgt gttcgt 46

<210> 14
<211> 47
<212> DNA
<213> Artificial

<220>
<223> An artificial primer of NS1.

<400> 14
cccgaattcc atatggtcga catggactcc aacaccgttt cctcctt 47

<210> 15
<211> 45
<212> DNA
<213> Artificial

<220>
<223>  An artificial primer of NS1.

<400>  15
ttcttccagg atacgttcaa cgatgtgttt accttcacgg gtagc                    45


<210>  16
<211>  43
<212>  DNA
<213>  Artificial

<220>
<223>  An artificial primer of NS1.

<400>  16
ggtcattttc agagcttcgt cggattcttc ttccaggata cgt                      43


<210>  17
<211>  43
<212>  DNA
<213>  Artificial

<220>
<223>  An artificial primer of NS1.

<400>  17
gtaacgcgga gccggaacgg aagcgatggt cattttcaga gct                      43


<210>  18
<211>  48
<212>  DNA
<213>  Artificial

<220>
<223>  An artificial primer of NS1.

<400>  18
acgggacatt tcttccaggg tcataccggt caggtaacgc ggagccgg                 48


<210>  19
<211>  54
<212>  DNA
<213>  Artificial

<220>
<223>  An artificial primer of NS1.

<400>  19
accggtaact ttctgtttcg ggatcagcat cagccagtca cgggacattt cttc          54


<210>  20
<211>  51
<212>  DNA
<213>  Artificial

<220>
<223>  An artificial primer of NS1.

<400>  20
attatttttc tcgaggtcca tacggatgca cagggaaccg gtaactttct g              51


<210>  21

<211>  42
<212>  DNA
<213>  Artificial

<220>
<223>  Artificial primer for fragment NA-a.

<400>  21
gctaccgaaa tccgtgcttc cgttggtcgt atgatctccg gt                    42


<210>  22
<211>  45
<212>  DNA
<213>  Artificial

<220>
<223>  Artificial primer of fragment NP-a.

<400>  22
cagatggaaa ccggtggtga acgtcagaac gctaccgaaa tccgt                 45


<210>  23
<211>  41
<212>  DNA
<213>  Artificial

<220>
<223>  Artificial primer of fragment NP-a.

<400>  23
tcccagggta ccaaacgttc ctacgaacag atggaaaccg g                     41


<210>  24
<211>  43
<212>  DNA
<213>  Artificial

<220>
<223>  Artificial primer of fragment NP-a.

<400>  24
aaagaattcg tcgaccatat gatggcttcc cagggtacca aac                   43


<210>  25
<211>  43
<212>  DNA
<213>  Artificial

<220>
<223>  Artificial primer of fragment NP-a.

<400>  25
gcacatctgg atgtagaaac gaccgatacc ggagatcata cga                   43


<210>  26
<211>  46
<212>  DNA
<213>  Artificial

<220>
<223>  Artifical primer of fragment NP-a.

<400>  26

gaccttcgta gtcggacagt ttcagttcgg tgcacatctg gatgta                    46

<210>  27
<211>  44
<212>  DNA
<213>  Artificial

<220>
<223>  Artificial primer of fragment NP-a.

<400>  27
ttcgtctttt ttggagttct gcatcagacg accttcgtag tcgg                    44

<210>  28
<211>  42
<212>  DNA
<213>  Artificial

<220>
<223>  Artificial primer of fragment NP-a.

<400>  28
gggctcgagt ttcagttcgt cacgcagttc gtcttttttg ga                    42

<210>  29
<211>  41
<212>  DNA
<213>  Artificial

<220>
<223>  Artificial primer of fragment NP-b.

<400>  29
tgggttcgtg aactgatcct gtacgacaaa gaagaaatcc g                    41

<210>  30
<211>  41
<212>  DNA
<213>  Artificial

<220>
<223>  Artificial primer of fragment NP-b.

<400>  30
ccgatctacc gtcgtcgtga cggtaaatgg gttcgtgaac t                    41

<210>  31
<211>  45
<212>  DNA
<213>  Artificial

<220>
<223>  Artificial primer of fragment NP-b.

<400>  31
gctggtaaag acccgaaaaa aaccggtggt ccgatctacc gtcgt                    45

<210>  32
<211>  42
<212>  DNA
<213>  Artificial

<220>
<223>  Artificial primer of fragment NP-b.

<400>  32
aaccgttacc tggaagaaca cccgtccgct ggtaaagacc cg                          42


<210>  33
<211>  42
<212>  DNA
<213>  Artificial

<220>
<223>  Artificial primer of fragment NP-b.

<400>  33
gttctgtccg ctttcgacga acgtcgtaac cgttacctgg aa                          42


<210>  34
<211>  42
<212>  DNA
<213>  Artificial

<220>
<223>  Artificial primer of fragment NP-b.

<400>  34
aaagaattcg tcgaccatat gcgtatggtt ctgtccgctt tc                          42


<210>  35
<211>  43
<212>  DNA
<213>  Artificial

<220>
<223>  An artificial primer of NP-b.

<400>  35
caccgttgtt agcctgacgc cagatacgac ggatttcttc ttt                         43


<210>  36
<211>  44
<212>  DNA
<213>  Artificial

<220>
<223>  Artificial primer of fragment NP-b.

<400>  36
caggtgggtc agaccagcgg tagcgtcttc accgttgtta gcct                        44


<210>  37
<211>  41
<212>  DNA
<213>  Artificial

<220>
<223>  Artificial primer of fragment NP-b.

<400>  37
tcgttcaggt tggagtgcca gatcatcagg tgggtcagac c                           41


<210>  38

<211>  42
<212>  DNA
<213>  Artificial

<220>
<223>  Artificial primer of fragment NP-b.

<400>  38
cacagcacgg gtacgctggt aggtagcgtc gttcaggttg ga                    42


<210>  39
<211>  46
<212>  DNA
<213>  Artificial

<220>
<223>  Artificial primer of fragment NP-b.

<400>  39
agcacatacg cgggtccata ccggtacgaa ccagagcacg ggtacg                46


<210>  40
<211>  44
<212>  DNA
<213>  Artificial

<220>
<223>  Artificial primer of fragment NP-b.

<400>  40
gggctcgagt ttcagttcgt ctttttggga gcacatacgc gggt                  44


<210>  41
<211>  44
<212>  DNA
<213>  Artificial

<220>
<223>  Artificial primer of fragment NP-c.

<400>  41
ctgaaaggta aattccagac cgctgctcag cgtgctatga tgga                  44


<210>  42
<211>  42
<212>  DNA
<213>  Artificial

<220>
<223>  Artificial primer of fragment NP-c.

<400>  42
atcgcttacg aacgtatgtg caacatcctg aaaggtaaat tc                    42


<210>  43
<211>  42
<212>  DNA
<213>  Artificial

<220>
<223>  Artificial primer of fragment NP-c.

<400>  43

cgtggtgaaa acggtcgtcg tacccgtatc gcttacgaac gt 42

<210> 44
<211> 42
<212> DNA
<213> Artificial

<220>
<223> Artificial primer of fragment NP-c.

<400> 44
cgtggtatcg acgaccgtaa cttctggcgt ggtgaaaacg gt 42

<210> 45
<211> 41
<212> DNA
<213> Artificial

<220>
<223> Artificial primer of fragment NP-c.

<400> 45
gttatggaac tgatccgtat gatcaaacgt ggtatcgacg a 41

<210> 46
<211> 41
<212> DNA
<213> Artificial

<220>
<223> Artificial primer of fragment NP-c.

<400> 46
gctgctgtta aaggtgttgg taccatggtt atggaactga t 41

<210> 47
<211> 42
<212> DNA
<213> Artificial

<220>
<223> Artificial primer of fragment NP-c.

<400> 47
ctgccgcgtc gttccggtgc tgctggtgct gctgttaaag gt 42

<210> 48
<211> 39
<212> DNA
<213> Artificial

<220>
<223> Artificial primer of fragment NP-c.

<400> 48
aaagaattcg tcgaccatta gaccctgccg cgtcgttcc 39

<210> 49
<211> 45
<212> DNA
<213> Artificial

<220>
<223>  Artificial primer of fragment NP-c.

<400>  49
agccaggaag atcagcagtt cgtctttttt gtccatcata gcacg                    45


<210>  50
<211>  41
<212>  DNA
<213>  Artificial

<220>
<223>  Artificial pr[?]      f fragment NP-c.

<400>  50
ggaac[?][?][?][?][?] [?][?]              [?]cgagc caggaagatc a                    41


<2[?]




<220>
<223>  Artificial prim[?][?] [?][?] [?]ragment NP-c.

[?][?][?]>  51
     [?]agg caggatt[?]       [?]a[?] gga accacgcagg atc                    43


<210>
<211>  [?]
<212>  D[?]
<213>  Ar[?][?][?][?][?][?]l

<220>
<223>  Artificial primer of fragment NP-c.

<400>  52
ggaagcaaca gccagaccgt aaacgcaagc cggcaggcag ga                    42


<210>  53
<211>  41
<212>  DNA
<213>  Artificial

<220>
<223>  Artificial primer of fragment NP-c.

<400>  53
gtaaccttca cgttcgaagt cgtaaccgga agcaacagcc a                    41


<210>  54
<211>  42
<212>  DNA
<213>  Artificial

<220>
<223>  Artificial primer of fragment NP-c.

<400>  54
acggaacggg tcgataccaa ccagggagta accttcacgt tc                    42


<210>  55

<211>  42
<212>  DNA
<213>  Artificial

<220>
<223>  Artificial primer of fragment NP-c.

<400>  55
ggagaaaacc tgggagtttt gcagcagacg gaacgggtcg at                    42


<210>  56
<211>  42
<212>  DNA
<213>  Artificial

<220>
<223>  Artificial primer of fragment NP-c.

<400>  56
gggctcgagt ttcagttcgt cacgcaggga gaaaacctgg ga                    42


<210>  57
<211>  41
<212>  DNA
<213>  Artificial

<220>
<223>  Artificial primer of fragment NP-d.

<400>  57
cgtaacctgc cgttcgaacg tgctaccatc atggctgttt t                     41


<210>  58
<211>  41
<212>  DNA
<213>  Artificial

<220>
<223>  Artificial primer of fragment NP-d.

<400>  58
gaagacaaaa aaaccttctc cgttcagcgt aacctgccgt t                     41


<210>  59
<211>  46
<212>  DNA
<213>  Artificial

<220>
<223>  Artificial primer of fragment NP-d.

<400>  59
gctatccgta cccgttccgg tggtaacctg gaagacaaaa aaacct                46


<210>  60
<211>  41
<212>  DNA
<213>  Artificial

<220>
<223>  Artificial primer of fragment NP-d.

<400>  60

accctggaac tgcgttcccg ttactccgct atccgtaccc g 41

<210> 61
<211> 42
<212> DNA
<213> Artificial

<220>
<223> Artificial primer of fragment NP-d.

<400> 61
gaaaacatgg aagctatgga ctccaacacc ctggaactgc gt 42

<210> 62
<211> 44
<212> DNA
<213> Artificial

<220>
<223> Artificial primer of fragment NP-d.

<400> 62
tccacccgtg gtgttcagat cgcttccaac gaaaacatgg aagc 44

<210> 63
<211> 45
<212> DNA
<213> Artificial

<220>
<223> Artificial primer of fragment NP-d.

<400> 63
ggtacccgtg ttgttccgcg tggtcagctg tccacccgtg gtgtt 45

<210> 64
<211> 46
<212> DNA
<213> Artificial

<220>
<223> Artificial primer of fragment NP-d.

<400> 64
aaagaattcg tcgaccatat gttcatccgt ggtacccgtg ttgttc 46

<210> 65
<211> 42
<212> DNA
<213> Artificial

<220>
<223> Artificial primer of fragment NP-d.

<400> 65
ggaggtacga ccttcggtgt taccggtgaa aacagccatg at 42

<210> 66
<211> 44
<212> DNA
<213> Artificial

<220>
<223>  Artificial primer of fragment NP-d.

<400>  66
catcatacgg atgatttcgg tacgcatgtc ggaggtacga cctt                        44


<210>  67
<211>  45
<212>  DNA
<213>  Artificial

<220>
<223>  Artificial primer of fragment NP-d.

<400>  67
gaaggaaacg tcttccggac gagcggattc catcatacgg atgat                       45


<210>  68
<211>  44
<212>  DNA
<213>  Artificial

<220>
<223>  Artificial primer of fragment NP-d.

<400>  68
gtcggacagt tcgaaaacac cacgaccctg gaaggaaacg tctt                        44


<210>  69
<211>  45
<212>  DNA
<213>  Artificial

<220>
<223>  Artificial primer of fragment NP-d.

<400>  69
ggacggaacg atcgggttgg tagctttttc gtcggacagt tcgaa                       45


<210>  70
<211>  41
<212>  DNA
<213>  Artificial

<220>
<223>  Artificial primer of fragment NP-d.

<400>  70
taggaacctt cgttgttcat gtcgaaggac ggaacgatcg g                           41


<210>  71
<211>  42
<212>  DNA
<213>  Artificial

<220>
<223>  Artificial primer of fragment NP-d.

<400>  71
gtattcttca gcgttgtcac cgaagaagta ggaaccttcg tt                          42


<210>  72

<211>  45
<212>  DNA
<213>  Artificial

<220>
<223>  Artificial primer of fragment NP-d.

<400>  72
gggctcgagt ttcagttcgt cacggttgta gtattcttca gcgtt                    45


<210>  73
<211>  45
<212>  DNA
<213>  Artificial

<220>
<223>  Artificial primer of fragment HA.

<400>  73
gttgtttggc tgatcaagaa aaacaacgct tacccgacca tcaaa                    45


<210>  74
<211>  45
<212>  DNA
<213>  Artificial

<220>
<223>  Artificial primer of fragment HA.

<400>  74
tacctgggta aatcctcctt cttccgtaac gttgtttggc tgatc                    45


<210>  75
<211>  46
<212>  DNA
<213>  Artificial

<220>
<223>  Artificial primer of fragment HA.

<400>  75
agcttcctcc ggtgtttctt ctgcttgccc gtacctgggt aaatcc                   46


<210>  76
<211>  42
<212>  DNA
<213>  Artificial

<220>
<223>  Artificial primer of fragment HA.

<400>  76
ccgaaatctt cctggtctaa ccacgaagct tcctccggtg tt                       42


<210>  77
<211>  45
<212>  DNA
<213>  Artificial

<220>
<223>  Artificial primer of fragment HA.

<400>  77

atcaaccact tcgaaaaaat ccagatcatc ccgaaatctt cctgg 45

<210> 78
<211> 46
<212> DNA
<213> Artificial

<220>
<223> Artificial primer of fragment HA.

<400> 78
ctacgaagaa ctgaaacacc tgctgtcccg tatcaaccac ttcgaa 46

<210> 79
<211> 42
<212> DNA
<213> Artificial

<220>
<223> Artificial primer of fragment HA.

<400> 79
ctgtgctacc cgggtgactt caacgactac gaagaactga aa 42

<210> 80
<211> 45
<212> DNA
<213> Artificial

<220>
<223> Artificial primer of fragment HA.

<400> 80
cccgaattcc atatggtcga cgctaacgac ctgtgctacc cgggt 45

<210> 81
<211> 45
<212> DNA
<213> Artificial

<220>
<223> Artificial primer of fragment HA.

<400> 81
gtcttcctgg ttggtgttgt tgtaggaacg tttgatggtc gggta 45

<210> 82
<211> 45
<212> DNA
<213> Artificial

<220>
<223> Artificial primer of fragment HA.

<400> 82
cgggtggtgg ataccccaca gaaccagcag gtcttcctgg ttggt 45

<210> 83
<211> 44
<212> DNA
<213> Artificial

<220>
<223>  Artificial primer of fragment HA.

<400>  83
tacagtttgg tctgttcagc agcgtcgttc gggtggtgga tacc                    44


<210>  84
<211>  48
<212>  DNA
<213>  Artificial

<220>
<223>  Artificial primer of fragment HA.

<400>  84
accaacggag atgtaggtgg tcgggttctg gtacagtttg gtctgttc                48


<210>  85
<211>  45
<212>  DNA
<213>  Artificial

<220>
<223>  Artificial primer of fragment HA.

<400>  85
cggaaccaga cgctggttca gggtggaggt accaacggag atgta                   45


<210>  86
<211>  45
<212>  DNA
<213>  Artificial

<220>
<223>  Artificial primer of fragment HA.

<400>  86
accgttaact ttggaacggg tagcgatttt cggaaccaga cgctg                   45


<210>  87
<211>  45
<212>  DNA
<213>  Artificial

<220>
<223>  Artificial primer of fragment HA.

<400>  87
ggtccagaag aactccatac gaccggactg accgttaact ttgga                   45


<210>  88
<211>  42
<212>  DNA
<213>  Artificial

<220>
<223>  Artificial primer of fragment HA.

<400>  88
attatttttc tcgagcggtt tcaggatggt ccagaagaac tc                       42


<210>  89

<211>  21
<212>  DNA
<213>  Artificial

<220>
<223>  Artificial primer of fragment M2.

<400>  89
atctaccgtc gtctgaaata c                                                21


<210>  90
<211>  42
<212>  DNA
<213>  Artificial

<220>
<223>  Artificial primer of fragment M2.

<400>  90
tgccgttgca gcgacagcag cgacccgatc taccgtcgtc tg                         42


<210>  91
<211>  39
<212>  DNA
<213>  Artificial

<220>
<223>  Artificial primer of fragment M2.

<400>  91
ccccgacccg taacgaatgg gaatgccgtt gcagcgaca                             39


<210>  92
<211>  66
<212>  DNA
<213>  Artificial

<220>
<223>  Artificial primer of fragment M2.

<400>  92
aaaaagaatt cgtcgaccat atgagcctgc tgaccgaagt tgaaaccccg acccgtaacg      60

aattgg                                                                 66


<210>  93
<211>  54
<212>  DNA
<213>  Artificial

<220>
<223>  Artificial primer of fragment M2.

<400>  93
tttcagaccg tatttcagac gacggtagat gcatttgaag aacagacggt ccag            54


<210>  94
<211>  52
<212>  DNA
<213>  Artificial

<220>
<223>  Artificial primer of fragment M2.

<400>  94
gcatgctttc cggaacacca gcggtgctcg gaccacgttt cagaccgtat tt                52

<210>  95
<211>  51
<212>  DNA
<213>  Artificial

<220>
<223>  Artificial primer of fragment M2.

<400>  95
agcgctctgc tgttcctgac ggtattcttc acgcatgctt tccggaacac c                 51

<210>  96
<211>  67
<212>  DNA
<213>  Artificial

<220>
<223>  Artificial primer of fragment M2.

<400>  96
tttctcgagt tcgatgttaa cgaagtgacc gtcgtcaacg tcaacagcgc tctgctgttc        60

ctgacgg                                                                  67

<210>  97
<211>  47
<212>  DNA
<213>  Artificial

<220>
<223>  Artificial primer of fragment NA.

<400>  97
ggttggaccg gtaccgactc ctccttctcc gttaaacagg acatcgt                      47

<210>  98
<211>  45
<212>  DNA
<213>  Artificial

<220>
<223>  Artificial primer of fragment NA.

<400>  98
tccggtttcg aaatgatctg ggacccgaac ggttggaccg gtacc                        45

<210>  99
<211>  45
<212>  DNA
<213>  Artificial

<220>
<223>  Artificial primer of fragment NA.

<400>  99
atcggtcgta ccaaatccac caactcccgt tccggtttcg aaatg                        45

<210>  100

<211>  47
<212>  DNA
<213>  Artificial

<220>
<223>  Artificial primer of fragment NA.

<400>  100
gtttctcctt caaatacggt aacggtgttt ggatcggtcg taccaaa                47


<210>  101
<211>  48
<212>  DNA
<213>  Artificial

<220>
<223>  Artificial primer of fragment NA.

<400>  101
atgtccctga acggtgctta cggtgttaaa ggtttctcct tcaaatac               48


<210>  102
<211>  45
<212>  DNA
<213>  Artificial

<220>
<223>  Artificial primer of fragment NA.

<400>  102
ccgaacgacg gtaccggttc ctgcggtccg atgtccctga acggt                  45


<210>  103
<211>  45
<212>  DNA
<213>  Artificial

<220>
<223>  Artificial primer of fragment NA.

<400>  103
tgctccggtg ttttcggtga caacccgcgt ccgaacgacg gtacc                  45


<210>  104
<211>  48
<212>  DNA
<213>  Artificial

<220>
<223>  Artificial primer of fragment NA.

<400>  104
cccgaattcc atatggtcga cggttacatc tgctccggtg ttttcggt               48


<210>  105
<211>  48
<212>  DNA
<213>  Artificial

<220>
<223>  Artificial primer of fragment NA.

<400>  105

accggagtaa ccggaccagt cggtgatagc aacgatgtcc tgtttaac 48

<210> 106
<211> 46
<212> DNA
<213> Artificial

<220>
<223> Artificial primer of fragment NA.

<400> 106
gaccggtcag ttccgggtgc tgaacgaagg aaccggagta accgga 46

<210> 107
<211> 48
<212> DNA
<213> Artificial

<220>
<223> Artificial primer of fragment NA.

<400> 107
ttcgatccag aagcacggac ggatgcagtc cagaccggtc agttccgg 48

<210> 108
<211> 45
<212> DNA
<213> Artificial

<220>
<223> Artificial primer of fragment NA.

<400> 108
ggtggattct ttcggacgac cacggatcag ttcgatccag aagca 45

<210> 109
<211> 46
<212> DNA
<213> Artificial

<220>
<223> Artificial primer of fragment NA.

<400> 109
agaaggagat ggaggaaccg gaggtccaga tggtggattc tttcgg 46

<210> 110
<211> 45
<212> DNA
<213> Artificial

<220>
<223> Artificial primer of fragment NA.

<400> 110
ccaaccaacg gtgtcggagt taacaccgca gaaggagatg gagga 45

<210> 111
<211> 44
<212> DNA
<213> Artificial

<220>
<223>  Artificial primer of fragment NA.

<400>  111
aacggcagtt cagcaccgtc cggccaggac caaccaacgg tgtc                    44


<210>  112
<211>  45
<212>  DNA
<213>  Artificial

<220>
<223>  Artificial primer of fragment NA.

<400>  112
attatttttc tcgagtttgt cgatggtgaa cggcagttca gcacc                    45